# EUROPEAN PATENT SPECIFICATION

(11) **EP 1 583 541 B1**
(45) Date of publication and mention of the grant of the patent: **12.01.2011**
(21) Application number: 03772495.2
(22) Date of filing: 20.11.2003
(51) Int. Cl.: A61K 38/26, A61K 31/675, A61P 25/00, A61K 38/23, A61K 38/27, A61K 38/22

(54) **COMPOUNDS AND METHODS FOR INCREASING NEUROGENESIS**
VERBINDUNGEN UND VERFAHREN ZUR ERHÖHUNG DER NEUROGENESE
COMPOSES ET METHODES POUR AUGMENTER LA NEUROGENESE

(30) Priority: 20.11.2002 US 427912 P
(43) Date of publication of application: 12.10.2005
(73) Proprietor: NeuroNova AB, 114 33 Stockholm (SE)
(72) Inventor: BERTILSSON, Göran, S-137 41 Västerhaninge (SE); ERLANDSSON, Rikard, S-174 59 Sundyberg (SE); FRISEN, Jonas, 114 26 Stockholm (SE); HAEGERSTRAND, Anders, S-182 56 Danderyd (SE); HEIDRICH, Jessica, S-120 50 Arsta (SE); HELLSTRÖM, Kristina, S-151 46 Södertälje (SE); HÄGGBLAD, Johan, S-118 28 Västgötagränd (SE); JANSSON, Katarina, S-121 45 Joanneshov (SE); KORTESMAA, Jarkko, S-118 28 Stockholm (SE); LINDQUIST, Per, S-175 63 Järfälla (SE); FRÄMME, Hanna, S-170 68 Solna (SE); MCGUIRE, Jacqueline, S-114 33 Stockholm (SE); MERCER, Alex, S-121 45 Johanneshov (SE); NYBERG, Karl, S-753 31 Uppsala (SE); OSSOINAK, Amina, S-113 38 Stockholm (SE); PATRONE, Cesare, S-126 49 Hägersten (SE); RÖNNHOLM, Harriet, 142 61 Trangsund (SE); ZACHRISSON, Olof, 163 54 Spanga (SE); WIKSTRÖM, Lilian, 163 54 Spanga (SE)
(74) Representative: Owen, Deborah Jane
(86) International application number: PCT/IB2003/005311
(87) International publication number: WO 2004/045592

(56) References cited:
- WO-A-00/12099
- WO-A-01/85981
- WO-A-2005/081619
- WO-A2-03/011892
- GRIMM-JORGENSEN Y: "SOMATOSTATIN AND CALCITONIN STIMULATE NEURITE REGENERATION OF MOLLUSCAN NEURONS IN-VITRO" BRAIN RESEARCH, vol. 403, no. 1, 1987, pages 121-126, XP008072630 ISSN: 0006-8993
- PERRY ET AL.: SOCIETY FOR NEUROSCIENCE ABSTRACTS, vol. 27, no. 1, 2001, page 298, XP008072635 31ST ANNUAL MEETING OF THE SOCIETY FOR NEUROSCIENCE; SAN DIEGO, CALIFORNIA, USA; NOVEMBER 10-15, 2001 ISSN: 0190-5295
- PERRY T ET AL: "A novel neurotrophic property of glucagon-like peptide 1: A promoter of nerve growth factor-mediated differentiation in PC12 cells" JOURNAL OF PHARMACOLOGY AND EXPERIMENTAL THERAPEUTICS, AMERICAN SOCIETY FOR PHARMACOLOGY AND, US, vol. 300, no. 3, March 2002 (2002-03), pages 958-966, XP002323789 ISSN: 0022-3565
- PERRY TRACYANN ET AL: "Protection and reversal of excitotoxic neuronal damage by glucagon-like peptide-1 and exendin-4" JOURNAL OF PHARMACOLOGY AND EXPERIMENTAL THERAPEUTICS, AMERICAN SOCIETY FOR PHARMACOLOGY AND EXPERIMENTAL THERAPEUTIC, US, vol. 302, no. 3, September 2002 (2002-09), pages 881-888, XP002402954 ISSN: 0022-3565
- HAILE C N ET AL: "COGNITIVE ENHANCEMENT AND NEUROPROTECTION THROUGH CENTRAL GLUCAGON - LIKE - 1 PEPTIDE ( GLP - 1 ) RECEPTORS." SOCIETY FOR NEUROSCIENCE ABSTRACT VIEWER AND ITINERARY PLANNER, vol. 2002, 2002, pages Abstract No. 384.21 URL-http://sf, XP008073036 & 32ND ANNUAL MEETING OF THE SOCIETY FOR NEUROSCIENCE; ORLANDO, FLORIDA, USA; NOVEMBER 02-07, 2002
- PERRY T ET AL: "GLUCAGON - LIKE PEPTIDE - 1 ( 7 - 36 ) - AMIDE ( GLP - 1 ) : NEW THERAPEUTIC STRATEGY FOR CENTRAL and PERIPHERAL NERVOUS SYSTEM DISORDERS." SOCIETY FOR NEUROSCIENCE ABSTRACT VIEWER AND ITINERARY PLANNER, vol. 2002, 2002, pages Abstract No. 786.12 URL-http://sf, XP008073035 & 32ND ANNUAL MEETING OF THE SOCIETY FOR NEUROSCIENCE; ORLANDO, FLORIDA, USA; NOVEMBER 02-07, 2002
- MILANI D ET AL: "Cross-linking of membrane-associated GM1 by B subunit of cholera toxin modulates intracellular calcium concentration and differentiation in sensory neurons" SOCIETY FOR NEUROSCIENCE ABSTRACTS, vol. 18, no. 1-2, 1992, page 411, XP008072730 & 22ND ANNUAL MEETING OF THE SOCIETY FOR NEUROSCIENCE; ANAHEIM, CALIFORNIA, USA; OCTOBER 25-30, 1992 ISSN: 0190-5295
- BRENNEMAN D E: "COMPARISON OF VASOACTIVE INTESTINAL PEPTIDE 10-28 PEPTIDE HISTIDINE ISOLEUCINE 27 SECRETIN AND GROWTH HORMONE-RELEASING FACTOR FOR EFFECTS ON NEURONAL SURVIVAL IN DEVELOPING SPINAL CORD CULTURES" SOCIETY FOR NEUROSCIENCE ABSTRACTS, vol. 12, no. 2, 1986, page 870, XP008072729 & 16TH ANNUAL MEETING OF THE SOCIETY FOR NEUROSCIENCE, PART 2, WASHINGTON, D.C., USA, NOV. 9-14, 1986. ISSN: 0190-5295
- OHTA SHIGERU ET AL: "Neuronal differentiation of Ewing's sarcoma induced by cholera toxin B and bromodeoxyuridine: Establishment of Ewing's sarcoma cell line and histochemical study" ACTA PAEDIATRICA JAPONICA, vol. 33, no. 4, 1991, pages 428-433, XP008072731 ISSN: 0374-5600
- GÖKE R. ET AL: 'Exendin-4 is a high potency agonist and truncated exendin-(9-39)-amide an antagonist at the glucagon-like peptide 1-(7-36)-amide receptor of insulin-secreting beta-cells' JOURNAL OF BIOLOGICAL CHEMISTRY, AMERICAN SOCIETY FOR BIOCHEMISTRY AND MOLECULAR BIOLOGY, INC, US vol. 268, no. 26, 15 September 1993, pages 19650 - 19655, XP002966930 ISSN: 0021-9258

## Description

### RELATED APPLICATIONS

This application claims the benefit of priority to U.S.S.N. 60/427,912 filed November 20, 2002.

### FIELD OF THE INVENTION

The invention is directed to in vitro and in vivo methods of modulating neurogenesis. Novel agents for increasing intracellular levels of cAMP, Ca²⁺ and for modulating neurogenesis are also described.

### BACKGROUND OF THE INVENTION

Studies performed in the 1960s provided the first indication that new neurons were produced in the adult mammalian brain (Altman and Das, 1965, 1967). However, it took another three decades, and the development of refined technical procedures, to disprove the dogma that mammalian neurogenesis was restricted to embryogenesis and the perinatal period (for review see Momma et al., 2000; Kuhn and Svendsen, 1999). Neural stem cells (NSC) are a source for new neurons in the mammalian CNS. NSC are located within the ependymal and/or subventricular zone (SVZ) lining the lateral ventricle (Doetsch et al., 1999; Johansson et al., 1999b) and in the dentate gyrus of the hippocampal formation (Gage et al., 1998). Recent studies reveal the potential for several additional locations of NSC within the adult CNS (Palmer et al., 1999). Asymmetric division of NSC maintains their starting number, while generating a population of rapidly dividing precursor, or progenitor cells (Johansson et al., 1999b). The progenitor cells respond to a range of cues that dictate the extent of their proliferation and their fate, both in terms of differentiation and positioning.

The NSC of the ventricular system in the adult are likely counterparts of the embryonic ventricular zone stem cells lining the neural tube. The progeny of these embryonic cells migrate away to form the CNS as differentiated neurons and glia (Jacobson, 1991). NSC persist in the adult lateral ventricle wall (LVW), generating neuronal progenitors that migrate down the rostral migratory stream to the olfactory bulb. There, they differentiate into granule cells and periglomerular neurons (Lois and Alvarez-Buylla, 1993). Substantial neuronal death occurs in the olfactory bulb, creating a need for continuous replacement of lost neurons which is satisfied by the migrating progenitors derived from the LVW (Biebl et al., 2000). In addition, there are indications that lost neurons from other brain regions can be replaced by progenitors from the LVW that differentiate into the phenotype of the lost neurons with appropriate neuronal projections and synapses with the correct target cell type (Snyder et al., 1997; Magavi et al., 2000).

*In vitro* cultivation techniques have been established to identify the external signals involved in the regulation of NSC proliferation and differentiation (Johansson et al., 1999b; Johansson et al., 1999a). The mitogens EGF and basic FGF allow cell culture expansion of neural progenitors isolated from the ventricle wall and the hippocampus (McKay, 1997; Johansson et al., 1999a). These dividing progenitors remain in an undifferentiated state, and grow into large clones of cells known as neurospheres. Upon the withdrawal of the mitogens and the addition of serum, the progenitors differentiate into neurons, astrocytes and oligodendrocytes, which are the three cell lineages of the brain (Doetsch et al., 1999; Johansson et al., 1999b). Specific growth factors can be added to alter the proportions of each cell type formed. For example, CNTF acts to direct the neural progenitors to an astrocytic fate (Johe et al., 1996; Rajan and McKay, 1998). The thyroid hormone, triiodothyronine (T3), promotes oligodendrocyte differentiation (Johe et al., 1996), while PDGF enhances neuronal differentiation by progenitor cells (Johe et al., 1996; William et al., 1997). Recently, it has been shown that indeed adult regenerated neurons are integrated into the existing brain circuitry, and contribute to ameliorating neurological deficits (Nakatomi et al., 2002). Interestingly, observations have also shown that neurogenesis is occurring not only at the level of the olfactory bulb and hippocampus. In this respect it has been suggested by Zhao et al, that this process can also occur in the adult mouse substantia nigra, opening up a new field of investigation for the treatment of Parkinson's disease (Zhao et al., 2003)

The ability to expand neural progenitors and manipulate their cell fate has enormous implications for transplant therapies for neurological diseases where specific cell types are lost. Parkinson's Disease (PD), for example, is characterised by degeneration of dopaminergic neurons in the substantia nigra. Previous transplantation treatments for PD patients have used fetal tissue taken from the ventral midbrain at a time when substantia nigra dopaminergic neurons are undergoing terminal differentiation (Herman and Abrous, 1994). These cells have been grafted onto the striatum where they form synaptic contacts with host striatal neurons, their normal synaptic target. This restores dopamine turnover and release to normal levels with significant functional benefits to the patient (Herman and Abrous, 1994) (for review see Bjorklund and Lindvall, 2000). However, the grafting of fetal tissue is limited by ethical considerations and a lack of donor tissue. The expansion and manipulation of adult NSC can potentially provide a range of well characterized cells for transplant-based strategies for neurodegenerative disease such as PD. To this aim, the identification of factors and pathways that govern the proliferation and differentiation of neural cell types is fundamentally important.

Studies have shown that intraventricular infusion of both EGF and basic FGF induces proliferation in the adult ventricle wall cell population. In the case of EGF, extensive migration of progenitors into the neighbouring striatal parenchyma has been observed (Graig et al., 1996; Kuhn et al., 1997). EGF increases differentiation into glial lineage and reduced the generation of neurons (Kuhn et al., 1997). Additionally, intraventricular infusion of BDNF in adult rats increases the number of newly generated neurons in the olfactory bulb and rostral migratory stream, and in parenchymal structures, including the striatum, septum, thalamus and hypothalamus (Pencea et al., 2001). Thus, several studies have shown that the proliferation of progenitors within the SVZ of the LVW can be stimulated and that their lineage can be guided to neuronal or glial fates. Yet, the number of factors known to affect neurogenesis *in vivo* is small and their effects are adverse or limited. Consequently, it is necessary to identify other factors that can selectively stimulate neural stem cell activity, proliferation of neural progenitors and effect differentiation into the target phenotype. Such factors can be used for in vivo stimulation of neurogenesis and the culturing of cells for transplantation therapy.

Ca²⁺ and cAMP represent important intracellular second messengers. Both can be activated following several external stimuli and it has shown to be activated by several G protein coupled receptors (GPCRs) (Neves et al., 2002). The cAMP cascade plays a role in neuronal survival and plasticity. Neuroepithelial cells have Ca²⁺ mobilization systems that can be activated mainly by the muscarinic receptor system during brain development.

### BRIEF SUMMARY OF THE INVENTION

One embodiment of the invention is directed to at least one agent that elevates intracellular cAMP levels in neural tissue, wherein said agent is selected from the group consisting of Thyrocalcitonin, Calcitonin, Glucagon-Like Peptide-1 (7-37), Exendin-3 and Exendin-4, and analogs of glucagon-like peptide-1 (7-37), Exendin-3 or Exendin-4, wherein said glucagon-Like peptide 1 (7-37), Extendin-3 or extendin-4 analog interacts with a 6-protein coupled receptor (GPCR) which is the Glucagon-like peptide 1 receptor, and a combination thereof, or a cAMP analog, wherein said cAMP analog is selected from the group consisting of 8-gCPT-2-O-Me-cAMP, 8-Br-cAMP, Rp-cAMPS, 8-Cl-cAMP, Dibutyryl cAMP, pCPT-cAMP, and N6-monobutyryladenosine 3',5-cyclic monophosphate, for use in increasing neurogenesis in neural tissue of a patient exhibiting a central nervous system disorder selected from the group consisting of neurodegenerative disorders, ischemic disorders, neurological traumas, and learning and memory disorders, wherein the agent increases neurogenesis in the patient, thereby increasing neurogenesis in the neural tissue of the patient, and wherein increasing neurogenesis is increasing proliferation, differentiation, migration or survival of an adult neural stern cell in said neural tissue. In the uses, one or more neurogenesis modulating agent is administered to the patient.

The neurogenesis modulating agent of the invention is selected from the group consisting of Thyrocalcitonin, Calcitonin, Glucagon-Like Peptide-1 (7-37), Exendin-3 and Exendin-4, and analogs thereof, and a combination thereof, or a cAMP analog, wherein said cAMP analog is selected from the group consisting of 8-pCPT-2-O-Me-cAMP, 8-Br-cAMP, Rp-cAMPS, 8-Cl-cAMP, Dibutyryl cAMP, pCPT-cAMP, and N6-monobutyryladenosine 3',5'-cyclic monophosphate. This disclosure also shows that an inhibitor of cAMP-specific phosphodiesterase, an activator of adenylate cyclase, and an activator of ADP-ribosylation of a stimulatory G protein can be used as neurogenesis modulating agents. These neurogenesis modulating agent are listed in the Detailed Description. The disorders that may be treated by the methods of the invention are also listed in the detailed description section and include, at least, Parkinson's disease and Parkinsonian disorders, Huntington's disease, Alzheimer's disease, multiple sclerosis, amyotrophic lateral sclerosis, Shy-Drager syndrome, progressive supranuclear palsy, Lewy body disease, spinal ischemia, ischemic stroke, cerebral infarction, spinal cord injury, and cancer-related brain and spinal cord injury, multi-infarct dementia, and geriatric dementia.

The level of administration may be at least 0.001 ng/kg/day, at least 0.01 ng/kg/day, 0.1 ng/kg/day, at least 1 ng/kg/day, at least 5 mg/kg/day, at least 10 mg/kg/day, or at least 50 mg/kg/day. In a preferred embodiment, the administration raises the intracellular levels of cAMP at least 20% above normal. The administration may lead to tissue concentrations of the agent of about 0.0001 nM to 50 nM.

Administration may be systemic or direct into the CNS of a patient. Other routes of administration include oral, subcutaneous, intraperitoneal, intramuscular, intracerebroventricular, intraparenchymal, intrathecal, intracranial, buccal, mucosal, nasal, and rectal administration or administration by a liposome delivery system.

In alternative uses described herein, the neurogenesis modulating agent elevates intracellular Ca²⁺ levels in a cell of a neural tissue of the patient. In such uses the neurogenesis modulating agent may be Amylin Receptor Antagonist/Calcitonin(8-32), ANP (human), CGRP (8-37), Endothelin-1 human, Bovine, Canine, Mouse, Porcine, Rat), g-MSH, Growth Hormone Releasing Factor, MGOP 27, PACAP-38 , Sarafotoxin S6a, Sarafotoxin S6b, Sarafotoxin S6c, Septide, Somatostatin-28, Cholera toxin from Vibrio Cholerae, Angiotensin II (human synthetic), [D-Pen2-5]-Enkephalin, Adrenomedullin, Endothelin-1 (human, Porcine,) and functional equivalents thereof.

Another embodiment of the invention is directed to an *in vitro* method of increasing cAMP levels in an adult neural stem cell, by administrating an effective amount of an agent selected from the group consisting of Thyrocalcitonin, Calcitonin, Glucagon-Like Peptide-1 (7-37), Exendin-3 and Exendin-4, and a combination thereof, to the cell. In this disclosure administering an agent to a cell comprises contacting a cell with an agent. Other cAMP elevating agents described herein may be Adrenocortico-tropic hormone, Endothelin-1 (human, porcine), MECA, HE-NECA, [Cys3,6, Tyr8, Pro9]-Substance P, [D-Arg0 Hyp3, Igl5, D-Igl7, Oic8]-Bradykinin, Adrenamedullin (human), [Des-Arg9, Leu8]-Bradylkinin, [Des-Arg9]-Bradykinin, [D-Pen2-5]-Enkephalin, [D-pGlu1, D-Phe2, D-Trp3,6]-LH-RH, Adrenomedullin (26-52), Adrenomedullin (22-52), a -Neo-Endorphin, b-MSH, a-MSH, CART (61-102), Cholecystokinin Octapeptide [CCK(26-33)], DTLET, DDAVP, Eledoisin, g-MSH, a-Neurokinin, PACAP-38, Beta-ANP, Galanin (1-13)-Spantide-Amide, M40, [Sar9, Met (0)11]-Substance P, Sarafotoxin S6a, Sarafotoxin S6b, Sarafotoxin S6c, [Nle8,18, Tyr34]-Parathyroid Hormone (1-34) Amide (Human), ACTH (Human), Urotensin II (Globy), Vasoactive Intestinal Peptide (Human, Porcine, Rat), Nor-Binaltorphimine, Agouti Related Protein (87-132)-Amide (Human) and a combination thereof. The cell may be in a patient, in which case the use is for stimulating intracelluar cAMP in a cell of a patient. The method of administration and the levels of administration may be any method or level discussed for neurogenesis modulating agents in this disclosure.

Another embodiment of the invention is directed to a method for increasing neurogenesis in vitro. In the method, a population of neural cells (comprising adult neural stem cells) is cultured. Then, at least one neurogenesis modulating agent is administered to the cell. The administration is repeated, if necessary, until a desired level of neurogenesis is achieved. In such methods increasing neurogenesis is increasing proliferation, differentiation, migration or survival of said adult neural stem cells, and the agent is selected from the group consisting of Thyrocalcitonin, Calcitonin, Glucagon-Like Peptide-1 (7-37), Exendin-3 and Exendin-4, and analogs of glucagon-like peptide-1 (7-37), Exendin-3 or Exendin-4, wherein said glucagon-Like peptide 1 (7-37), Extendin-3 or extendin-4 analog interacts with a 6-protein coupled receptor (GPCR) which is the Glucagon-like peptide 1 receptor, and a combination thereof, or a cAMP analog, wherein said cAMP analog is selected from the group consisting of 8-pCPT-2-0-Me-cAMP, 8-Br-cAMP, Rp-cAMPS, 8-Cl-cAMP, Dibutyryl cAMP, pCPT-cAMP, and N6-monobutyryladenosine 3',5'-cyclic monophosphate, The adult neural stem cell may be cultured from tissue such as cortex, olfactory tubercle, retina, septum, lateral ganglionic eminence, medial ganglionic eminence, amygdala, hippocampus, thalamus, hypothalamus, ventral and dorsal mesencephalon, brain stem, cerebellum, spinal cord.

This disclosure also shows a role for G-protein coupled receptors (GPCRs) and their ligands in stem cells biology *in vitro* and *in vivo.* The invention is based on our expression data (PCR and cDNA library data) and *in vitro* proliferation data, which shows that modulation of intracellular cAMP or Ca²⁺ levels through various GPCRs can be used to influence proliferation, migration, differentiation or survival of adult neural stem cells (aNSC) and their progeny in *vitro* as well as *in situ* in the intact brain. This data also indicates CREB as a downstream link between GPCRs and transcription.

In all cases, the cell, neural tissue, or patient may be any adult mammal such as rat, mice, cat, dog, horse, pig, goat, cow and in particular human.

### BRIEF DESCRIPTION OF THE FIGURES

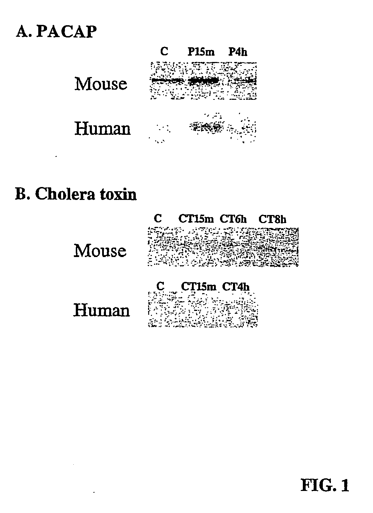

**FIGURE 1****:** CREB phosphorylation following PACAP and cholera toxin treatment occus in a reproducible manner in both mouse and human adult neural stem cells as shown by Western blotting. The upper panel shows up-regulation of CREB phosphorylation in mouse and human adult neural stem cells after PACAP treatment. The lower panel shows up-regulation of CREB phosphorylation in both mouse and human adult neural stern cells after cholera toxin treatment.

### DETAILED DESCRIPTION OF THE INVENTION

Traditional treatments of neural diseases and injuries have focused on the prevention of neuronal death (i.e., apoptosis or necrosis). In contrast, this invention is directed to novel therapeutic treatments for neurological diseases and injuries based on inducing neurogenesis, in particular, adult neural stem cell proliferation. In accordance with the invention, key neurogenesis modulating agents have been identified to induce proliferation and/or differentiation in adult neural stem cells. Such neurogenesis modulating agents are useful for effecting neurogenesis for the treatment of neurological diseases and injuries. As shown herein, increased levels of cAMP and/or Ca²⁺ elicit the proliferation of adult neural stem cells. In some cases, this induction follows the activation of G-protein coupled receptors (GPCRs). The data disclosed herein indicate that increasing intracellular cAMP and/or Ca²⁺ levels through various compounds (e.g., GPCR ligands) can be used to increase proliferation of adult neural stem cells. Furthermore, the data indicates that the progeny of the cells induced to proliferate by all the compounds analysed, also retain their full neurogenic potential. Expression data for the GPCRs that bind to these ligands corroborate the importance of these two second messengers in promoting neurogenesis.

"Neurogenesis" is defined herein as proliferation, differentiation, migration or survival of a neural cell *in vivo* or *in vitro.* In the present invention, the neural cell is an adult neural stem cell. Neurogenesis also refers to a net increase in cell number or a net increase in cell survival. As used herein, "NSC" would include, at least, all brain stem cells, all brain progenitor cells, and all brain precursor cells.

A neurogenesis modulating agent is defined as an agent or reagent that can promote neurogenesis. A number of novel neurogenesis modulating agents are disclosed in this invention.

In this disclosure, the terms disease or disorder shall have the same meaning.

All the methods of the invention may be used on mammals and mammalian cells. In a preferred embodiment, all the methods of the invention may be used on humans or human Cells.

Neural tissue includes, at least, all the tissues of the brain and central nervous system.

Neurobiologists have used various terms interchangeably to describe the undifferentiated cells of the CNS. Terms such as "stem cell", "precursor cell" and "progenitor cell" are commonly used in the scientific literature. However, there are different types of undifferentiated cells, with differing characteristics and fates. The capability of a cell to divide without limit and produce daughter cells which terminally differentiate into neurons and glia are stem cell characteristics. Thus, the term "stern cell" (e.g., neural stem cell), as used herein, refers to an undifferentiated cell that can be induced to proliferate using the methods of the present invention. The stem cell is capable of self-maintenance, meaning that with each cell division, one daughter cell will also be a stem cell. The non-stem cell progeny of a stem cell are termed progenitor cells. The progenitor cells generated from a single multipotent stem cell are capable of differentiating into neurons, astrocytes (type I and type II) and oligodendrocytes. Hence, the stem cell is multipotent because its progeny have multiple differentiative pathways.

The term " progenitor cell" (e.g., neural progenitor cell), as used herein, refers to an undifferentiated cell derived from a stem cell, and is not itself a stem cell. Some progenitor cells can produce progeny that are capable of differentiating into more than one cell type. For example, an O-2A cell is a glial progenitor cell that gives rise to oligodendrocytes and type II astrocytes, and thus could be termed a bipotential progenitor cell. A distinguishing feature of a progenitor cell is that, unlike a stem cell, it has limited proliferative ability and thus does not exhibit self-maintenance. It is committed to a particular path of differentiation and will, under appropriate conditions, eventually differentiate into glia or neurons. The term "precursor cells", as used herein, refers to the progeny of stem cells, and thus includes both progenitor cells and daughter stem cells.

### 1. Neurogenesis modulating agents

Described herein are neurogenesis modulating agents that modulate intracellular levels of cAMP and/or Ca²⁺. As used herein, neurogenesis modulating agent also include any substance that is chemically and biologically capable of increasing cLAMP (e.g., by increasing synthesis or decreasing breakdown) and/or Ca²⁺ (e.g., by increasing influx or decreasing efflux). These neurogenesis modulating agents include peptides, proteins, fusion proteins, chemical compounds and small molecules e.g. Thyrocalcitonin, Calcitonin, Glucagon-Like Peptide-1 (7-37), Exendin-3 and Exendin-4, and analogs thereof and a combination thereof, or a cAMP analog, wherein said cAMP analog is selected from the group consisting of 9-pCPT-2-O-Me-cAMP, 8-Br-cAMP, Rp-cAMPS, 8-Cl-cAMP, Dibutyryl cAMP, pCPT-cAMP, and N6-monobutyryladenosine 3',5'-cyclic monophosphate, PDE inhibitors (e.g., cAMP-specific PDEs), adenylate cyclase activators, and activators of ADP-ribosylation of stimulatory G proteins are further examples of neurogenesis modulating agents described herein.

Analogs of cAMP are: 8-pCPT-2-O-Me-cAMP (e.g., 8-(4-chlorophenylthio)-2'-O-methyladenosine 3',5'-cyclic monophosphate); 8-Br-cAMP (e.g., 8-bromoadenosine 3',5'-cyclic monophosphate); Rp-cAMPS (e.g., Rp-adenosine 3',5'-cyclic monophosphorothioate); 8-Cl-cAMP (e.g., 8-chloroadenosine 3',5'-cyclic-monophosphate); Dibutyryl cAMP (e.g., N6,2'-O-dibutyryladenosine 3',5'-cyclic monophosphate); pCPT-cAMP (e.g., 8-(4-chlorophenylthio)adenosine 3',5'-cyclic monophosphate); and N6-monobutyryladenosine 3',5'-cyclic monophosphate.

Exemplary PDE inhibitors include: theophylline (e.g., 3,7-dihydro-1,3-dimethyl-1H-purine-2,6-dione; 2,6-dihydroxy-1,3-dimethylpurine; 1,3-dimethylxanthine), caffeine (e.g., 1,3,7-trimethylxanthine); quercetin dihydrate (e.g., 2-(3,4-dihydroxyphenyl)-3,5,7-trihydroxy-4H-1-benzopyran-4-one dihydrate; 3,3',4',5,7-pentahydroxyflavone dihydrate); rolipram (e.g., 4-[3-(cyclopentyloxy)-4-methoxyphenyl]-2-pyrrolidinone); 4-(3-butoxy-4-methoxybenzyl)imidazolidin-2-one; propentofylline (e.g., 3,7-dihydro-3-methyl-1-(5-oxobexyl)-7-propyl-1H-purine-2,6-dione; 3-methyl-1-(5-oxohexyl)-7-propylxanthine); 3-jsobutyl-1-methylxanthine (e.g., 3,7-dihydro-1-methyl-3-(2-methylpropyl)-1H-purine-2,6-dione; IBMX; 3-isobutyl-1-methyl-2,6(1H,3h)-purinedione; 1-methyl-3-isobutylxanthine); 8-Methoxymethyl-3-isobutyl-1-methylxanthine (e.g., 8-methoxymethyl-IBMX); enoximone (e.g., 1,3-dihydro-4-methyl-5-[4-methylthiobenzoyl]-2H-imidazol-2-one); papaverine hydrochloride (e.g., 6,7-Dimethoxy 1-veratrylisoquinoline hydrochloride).

Other exemplary PDE inhibitors include: calmidazolium chloride (e.g. 1-[bis(4-chlorophenyl)methyl]-3-[2,4-dichloro-b-(2,4-dichlorobenzyloxy)phenethyl] imidazolium chloride; 1-[bis(4-chlorophenyl)methyl]-3-[2-(2,4-dichlorophenyl)-2-(2,4-dichlorobenzyloxy)ethyl]-1H-imidazolium chloride); SKF 94836 (e.g., N-cyano-N'-methyl-N"-[4-(1,4,5,6-tetrahydro-4-methyl-6-oxo-3-pyridazinyl)phenyl]guanidine; Siguazodan); neuropeptide Y fragment 22-36 (e.g., Ser-Ala-Leu-Arg-His-Tyr-Ile-Asn-Leu-Ile-Thr-Arg-Gln-Arg-Tyr; aminophylline hydrate (e.g., 3,7-Dihydro-1,3-dimethyl-1H-purine-2,6-dione, compound with 1,2-ethanediamine (2:1)(Theophylline)2; ethylenediamine; theophylline hemiethylenediamine complex); butein (e.g., 1-(2,4-dihydroxyphenyl)-3-(3,4-dihydroxyphenyl)-2-propen-1-one; 2',3,4,4'-tetrahydroxychalcone); papaverine hydrochloride (e.g., 6,7-dimethoxy-1-veratrylisoquinoline hydrochloride); etazolate hydrochloride (e.g., 1-ethyl-4-[(1-methylethylidene)hydrazino]1H-pyrazolo[3,4-b]pyridine-5-carboxilic acid ethyl ester hydrochloride); trifluoperazine dihydrochloride (e.g., 10-[3-(4-methyl-1-piperazinyl)propyl]-2-trifluoromethyl-phenothiazine dihydrochloride; trifluoroperazine dihydrochloride); and milrinone (e.g., 1,6-Dihydro-2-methyl-6-oxo-(3,4'-bipyridine)-5-carbonitrile).

Exemplary stimulators of ADP ribosylation include: Pertussis toxin (e.g., Pertussigen from *Borderella pertussis;* Histamine-sensitizing factor; IAP; Islet Activating Protein); and Cholera toxin (e.g., Cholergen from *Vibrio cholera,* Cholera enterotoxin).

Exemplary activators of adenylate cyclase include: forskolin.

Agents that have been shown in the experiments detailed herein to increase intracellular levels of cAMP include

| **Name** | **Peptide sequence** | **SEQ ID NO:** |
|---|---|---|
| Nor-binaltorphimine | Na | |
| | | SEQ ID NO:1 |
| PACAP-38 | His-Ser-Asp-Gly-Ile-Phe-Thr-Asp-Ser-Tyr-Ser-Arg-Tyr-Arg- Lys-Gln-Met-Ala-Val-Lys-Lys-Tyr-Leu-Ala-Ala-Val-Leu-Gly- Lys-Arg-Tyr-Lys-Gln-Arg-Val-Lys-Asn-Lys-NH2 | |
| | | SEQ ID NO:2 |
| Endothelin 1, human, porcine | Cys-Ser-Cys-Ser-Ser-Leu-Met-Asp-Lys-Glu-Cys-Val-Tyr- Phe-Cys-His-Leu-Asp-Ile-Ile-Trp | |
| | | SEQ ID NO:3 |
| Adrenocorticotropic Hormone | | |
| a-Melanacyte Stimulating Hormone | Ser-Tyr-Ser-Met-Glu-His-Phe-Arg-Trp-Gly-Lys-Pro-Val-Gly- Lys-Lys-Arg-Arg-Pro-Val-Lys-Val-Tyr-Pro Ac-Ser-Tyr-Ser-Met-Glu-His-Phe-Arg-Trp-Gly-Lys-Pro-Val- NH2 | SEQ ID NO:4 |
| g-Melanocyte stimulating hormone | Tyr-Val-Met-Gly-Hls-Phe-Arg-Trp-Asp-Arg-Phe-Gly | SEQ ID NO:5 |
| a-Neurokinin | His-Lys-Thr-Asp-Ser-Phe-Val-Gly-Leu-Met-NH2 | SEQ ID NO:6 |
| Thyrocalcitonin salmon | Cys-Ser-Asn-Leu-Ser-Thr-Cys-Val-Leu-Gly-Lys-Leu-Ser- Gln-Glu- Leu-His-Lys-Leu-Gln-Thr-Tyr-Pro-Arg-Thr-Asn-Thr- Gly-Ser-Gly- Thr-Pro-NH2 | SEQ ID NO:7 |
| b-Melanocyte stimulating hormone (beta-MSH), human | Ala-Glu-Lys-Lys-Asp-Glu-Gly-Pro-Tyr-Arg-Met-Glu-His-Phe Arg- Trp-Gly-Ser-Pro-Pro-Lys-Asp | SEQ ID NO:6 |
| MECA | non-peptide | |
| HE-NECA, 2-hexynyl-5 n-ethylcarboxamido | non-peptide | |
| [Cys3,6, Tyr8, Pro9]-Substance P (selective Neurokinin-1 agonist) | Arg-Pro-Cys-Pro-Gln-Cys-Phe-Tyr-Pro-Leu-Met | SEQ ID NO:9 |
| [Des-Arg9, Leu8]-Bradykinin(B1 antagonist) | Arg-Pro-Pro-Gly-Phe-Ser-Pro-Leu | SEQ ID NO:10 |
| [Des-Arg9]-Bradykinin(B1 antagonist) | Arg-Pro-Pro-Gly-Phe-Ser-Pro-Phe | SEQ ID NO:11 |
| [D-Pen2-5]-Enkephalin (Potent delta agonist) | Tyr-D-Pen-Gly-Phe-D-Pen | SEQ ID NO:12 |
| [D-pGlu1, D-Phe2, D-Trp3,6]-LH-RH | D-pGlu-D-Phe-D-Trp-Ser-Tyr-D-Trp-Leu-Arg-Pro-Gly-NH2 | SEQ ID NO:13 |
| | | SEQ ID NO:14 |
| [Nle8, 18, Tyr34]-Parathyroid Hormone (1-34) Amide (Human) | Ser-Val-Ser-Glu-Ile-Gln-Leu-Nle-His-Asn-Leu-Gly-Lys-His- Leu-Asn-Ser-Nle-Glu-Arg-Val-Glu-Trp-Leu-Arg-Lys-Lys-Leu- Gln-Asp-Val-His-Asn-Tyr | |
| | | SEQ ID NO:15 |
| ACTH (Human) | Ser-Tyr-Ser-Met-Glu-His-Phe-Arg-Trp-Gly-Lys-Pro-Val-Gly- Lys- Lys-Arg-Arg-Pro-Val-Lys-Val-Tyr-Pro-Asn-Gly-Ala-Glu- Asp-Glu- Ser-Ala-Glu-Ala-Phe-Pro-Leu-Glu-Phe | |
| | | SEQ ID NO:16 |
| Adrenomedullin (Human) | Tyr-Arg-Gln-Ser-Met-Asn-Asn-Phe-Gln-Gly-Leu-Arg-Ser- Phe-Gly- Cys-Arg-Phe-Gly-Thr-Cys-Thr-Val-Gln-Lys-Leu- Ala-His-Gln-Ile- Tyr-Gln-Phe-Thr-Asp-Lys-Asp-Lys-Asp-Asn- Val-Ala-Pro-Arg-Ser- Lys-Ile-Ser-Pro-Gln-Gly-Tyr-NH2 | |
| | | SEQ ID NO:17 |
| Adrenomedullin (22-52) (Human) | Thr-Val-Gln-Lys-Leu-Ala-His-Gln-Ile-Tyr-Gln-Phe-Thr-Asp- Lys- Asp-Lys-Asp-Asn-Val-Ala-Pro-Arg-Ser-Lys-Ile-Ser-Pro- Gln-Gly- Tyr-NH2 | |
| Adrenomedullin (26-52) | | SEQ ID NO:18 |
| (Human)(ADM antagonist) | Leu-Ala-His-Gln-Ile-Tyr-Gln-Phe-Thr-Asp-Lys-Asp-Lys-Asp- Asn- Val-Ala-Pro-Arg-Ser-Lys-Ile-Ser-Pro-Gln-Gly-Tyr-NH2 | |
| Alpha-Neo-Endorphin (Porcine) | | SEQ ID NO:19 |
| Beta-ANP (Human) | Tyr-Gly-Gly-Phe-Leu-Arg-Lys-Tyr-Pro-Lys Ser-Leu-Arg-Arg-Ser-Ser-Cys-Phe-Gly-Gly-Arg-Met-Asp- Arg-Ile- Gly-Ala-Gln-Ser-Gly-Leu-Gly-Cys-Asn-Ser-Phe-Arg- Tyr | SEQ ID NO:20 |
| | | SEQ ID NO:21 |
| Calcitonin (Human) | Cys-Gly-Asn-Leu-Ser-Thr-Cys-Met-Leu-Gly-Thr-Tyr-Thr- Gln-Asp-Phe-Asn-Lys-Phe-His-Thr-Phe-Pro-Gln-Thr-Ala-Ile- Gly-Val-Gly-Ala-Pro | |
| | | SEQ ID NO:22 |
| CART (61-102)(Human, Rat) | Lys-Tyr-Gly-Gln-Val-Pro-Met-Cys-Asp-Ala-Gly-Glu-Gln-Cys Ala-Val-Arg-Lys-Gly-Ala-Arg-Ile-Gly-Lys-Leu-Cys-Asp-Cys- Pro-Arg-Gly-Thr-Ser-Cys-Asn-Ser-Phe-Leu-Leu-Lys-Cys- Leu | |
| Cholecystokinin Octapeptide [CCK(26-33)](Non-sulfated) | Asp-Tyr-Met-Gly-Trp-Met-Asp-Phe-NH2 | SEQ ID NO:23 |
| DDAVP (enhances human learning and memory) | Mpr-Tyr-Phe-Gln-Asn-Cys-Pro-D-Arg-Gly-NH2 | SEQ ID NO:24 |
| DTLET | Tyr-D-Thr-Gly-Phe-Leu-Thr | SEQ ID NO:25 |
| | | SEQ ID NO:26 |
| Eledoisin | Glu-Pro-Ser-Lys-Asp-Ala-Phe-Ile-Gly-Leu-Met | |
| | | SEQ ID NO:27 |
| Galanin (1-13)-Spantide-Amide, M40 | Gly-Trp-Thr-Leu-Asn-Ser-Ala-Gly-Tyr-Leu-Leu-Gly-Pro-Pro- Pro- Ala-Leu-Ala-Leu-Ala-NH2 | |
| | | SEQ ID NO:28 |
| Glucagon-Like Peptide-1 (7-37) (Human) | His-Ala-Glu-Gly-Thr-Phe-Thr-Ser-Asp-Val-Ser-Ser-Tyr-Leu- Glu- Gly-Gln-Ala-Ala-Lys-Glu-Phe-Ile-Ala-Trp-Leu-Val-Lys- Gly-Arg- Gly | |
| | | SEQ ID NO:29 |
| Exendin-3 | His-Ser-Asp-Gly-Thr-Phe-Thr-Ser-Asp-Leu-Ser-Lys-Gln- Met-Glu- Glu-Glu-Ala-Val-Arg-Leu-Phe-Ile-Glu-Trp-Leu-Lys- Asn-Gly-Gly- Pro-Ser-Ser-Gly-Ala-Pro-Pro-Pro-Ser-NH2 | |
| | | SEQ ID NO:30 |
| Exendin-4 | His-Gly-Glu-Gly-Thr-Phe-Thr-Ser-Asp-Leu-Ser-Lys-Gln-Met- Glu- Glu-Glu-Ala-Val-Arg-Leu-Phe-Ile-Glu-Trp-Leu-Lys-Asn- Gly-Gly- Pro-Ser-Ser-Gly-Ala-Pro-Pro-Pro-Ser-NH2 | |
| [Sar9, Met(O)11]-Susbstance P (Highly selective NK-1 receptor agonist) | Arg-Pro-Lys-Pro-Gln-Gln-Phe-Phe-Sar-Leu-Met(O2)-NH2 | SEQ ID NO:31 |
| | | SEQ ID NO:32 |
| Sarafotoxin S6a (cardiotoxin isotoxin) | Cys-Ser-Cys-Lys-Asp-Met-Thr-Asp-Lys-Glu-Cys-Leu-Asn- Phe-Cys- His-Gln-Asp-Val-Ile-Trp | |
| Sarafotoxin S6b (potent coronary constrictor activity) | Cys-Ser-Cys-Lys-Asp-Met-Thr-Asp-Lys-Glu-Cys-Leu-Tyr- Phe-Cys- His-Gln-Asp-Val-Ile-Trp | SEQ ID NO:33 |
| Sarafotoxin S6c (cause strong vasoconstriction of coronary vessels) | Cys-Thr-Cys-Asn-Asp-Met-Thr-Asp-Glu-Glu-Cys-Leu-Asn- Phe-Cys- His-Gln-Asp-Val-Ile-Trp | SEQ ID NO:34 |
| Urotensin II (Globy) | | SEQ ID NO:35 |
| Vasoactive Intestinal Peptide (Human, Porcine, Rat) | Ala-Gly-Thr-Ala-Asp-Cys-Phe-Trp-Lys-Tyr-Cys-Val His-Ser-Asp-Ala-Val-Phe-Thr-Asp-Asn-Tyr-Thr-Arg-Leu-Arg- Lys- Gln-Met-Ala-Val-Lys-Lys-Tyr-Leu-Asn-Ser-Ile-Leu-Asn- NH2 | SEQ ID NO:36 |
| [D-Arg0, Hyp3, Igl5, D-Igl7, Olc8]-Bradykinin | | SEQ ID NO:37 |
| (B1/B2 Antagonist) | D-Arg-Arg-Pro-Hyp-Gly-Igl-Ser-D-Igl-Oic-Arg | |
| | | SEQ ID NO:38 |
| Agouti Signalling Protein (ASP)(87-132) Amide (Human) | Thr-Pro-Leu-Ser-Ala-Pro-Cys-Val-Ala-Thr-Arg-Asn-Ser-Cys- Lys- Pro-Pro-Ala-Pro-Ala-Cys-Cys-Asp-Pro-Cys-Ala-Ser- Cys-Gln-Cys- Arg-Phe-Phe-Arg-Ser-Ala-Cys-Ser-Cys-Arg- Val-Leu-Ser-Leu-Asn- Cys-NH2 | |
| | | SEQ ID NO:39 |
| Agoutl Related Protein (87-132)-Amide (Human) | Arg-Cys-Val-Arg-Leu-His-Glu-Ser-Cys-Leu-Gly-Gln- Gln- Val-Pro-Cys-Cys-Asp-Pro-Cys-Ala-Thr-Cys-Tyr-Cys-Arg- Phe- Phe-Asn-Ala-Phe-Cys-Tyr-Cys-Arg-Lys-Leu-Gly-Thr- Ala-Met-Asn- Pro-Cys-Ser-Arg-Thr-NH2 | |

Other agents which can increase intracellular cAMP include fenoldopam methanesulphonate, dopamine hydrochloride, apomorphine hydrochloride, histamine phosphate, ACTH, sumatriptan succinate, prostaglandin F2alpha tromethamine, prostaglandin E1, prostaglandin I2, iloprost tromethamine, prostaglandin E2, misoprostol, sulproston, ATP disodium salt, pindolol, secretin, cisapride, phentolamine methanesulphonate, nemonapride, clozapine, sertindole, olanzapine, risperidone, sulpiride, levosulpride,Chlorpromazine, chlorpromazine, hydrochloride, haloperidol, domperidone, fluphenazine dihydrochloride/decanoate/enantate, fluphenazine" dihydrochloride/decanoate, fluphenazine dihydrochloride, ATP (adenosin triphosphate), ATP (adenosin triphosphate) disodium salt, ketanserin,ketanserin tartare, metergoline, pindolol, prazosin hydrochloride, Yohimbine, yohimbine hydrochloride, theophylline, caffeine, theobromine, aminophylline, amrinone, milrinone, naltrexone, naloxone, albuterol, levalbuterol, metaproterenol, terbutaline, pirbuterol, salmeterol, bitolterol, colterol, dobutamine, 8L-arginine-vasopressin, 8-lysine-vasopressin, desmopressin, methyldopa, DOPA, rauwolshine, prazosin, phentolamine, quinidine, dapiprazole, loxiglumide, chorionic gonadotropin, follitropin-alpha, follitropin-beta(FSH), menotropin (LH, FSH), oxytocin, somatostatin antagonists, RMP-7, ACE inhibitors (like captopril), misoprostol, latanoprost, PGE1, alprostadil, somatropin (GH, PRL) secretagogues (MK-677), tabimorelin (NN-703, pamorelin, NNC-26-0323, TRH, cosyntropin, corticorelin, glucagon, enteroglucagon, PTH 1-34, cocaine, amphetamine. dextroamphetamine, metamphetamien, phenmetrazine, methylphenidate, diethylpropion, metyrosine, reserpine, minoxidil, sulfasalazine, levamisole, and thalidomide, fluoride.

Exemplary agents for increasing intracellular Ca²⁺ levels include the agents summarized in the table below:

| **Name** | **Peptide sequence** | |
|---|---|---|
| PACAP-38 | His-Ser-Asp-Gly-Ile-Phe-Thr-Asp-Ser-Tyr-Ser-Arg-Tyr- Arg-Lys-Gln-Met-Ala-Val-Lys-Lys-Tyr-Leu-Ala-Ala-Val- Leu-Gly-Lys-Arg-Tyr-Lys-Gln-Arg-Val-Lys-Asn-Lys- NH2 | SEQ ID NO:1 |
| Cholera toxin from Vibrio | | |
| Cholerae | non-peptide | |
| Endothelin 1, human, porcine | Cys-Ser-Cys-Ser-Ser-Leu-Met-Asp-Lys-Glu-Cys-Val- Tyr-Phe-Cys- His-Leu-Asp-Ile-Ile-Trp | SEQ ID NO:2 |
| Angiotensin II, human syntetic | Asp-Arg-Val-Tyr-Ile-His-Pro-Phe | SEQ ID NO:40 |
| g-Melanocyte stimulating hormone | Tyr-Val-Met-Gly-His-Phe-Arg-Trp-Asp-Arg-Phe-Gly Ser-Leu-Arg-Arg-Ser-Ser-Cys-Phe-Gly-Gly-Arg-Met- Asp-Arg-Ile-Gly-Ala-Gln-Ser-Gly-Leu-Gly-Cys-Asn- Ser-Phe-Arg-Tyr | SEQ ID NO:5 SEQ ID NO:41 |
| Atrial Natriuretic peptide, human | | |
| [D-Pen2-5]-Enkephalin | | SEQ ID NO:42 |
| (Potent delta agonist) | | |
| Adrenomedullin (Human) | Tyr-D-Pen-Gly-Phe-D-Pen Tyr-Arg-Gln-Ser-Met-Asn-Asn-Phe-Gln-Gly-Leu-Arg- Ser-Phe-Gly- Cys-Arg-Phe-Gly-Thr-Cys-Thr-Val-Gln- Lys-Leu-Ala-His-Gln-Ile- Tyr-Gln-Phe-Thr-Asp-Lys- Asp-Lys-Asp-Asn-Val-Ala-Pro-Arg-Ser- Lys-Ile-Ser- Pro-Gln-Gly-Tyr-NH2 | SEQ ID NO:43 |
| Amylin Receptor Antagonist/Calcitonin(8-32)(Salmon) | Val-Leu-Gly-Lys-Leu-Ser-Gln-Glu-Leu-His-Lys-Leu- Gln-Thr-Tyr- Pro-Arg-Thr-Asn-Thr-Gly-Ser-Gly-Thr-Pro | SEQ ID NO:44 |
| CGRP (8-37) (Human) | | SEQ ID NO:45 |
| (Selective antagonist for CGRP receptor and agonist for Calcitonin receptor) | Val-Thr-His-Arg-Leu-Ala-Gly-Leu-Leu-Ser-Arg-Ser- Gly-Gly-Val- Val-Lys-Asn-Asn-Phe-Val-Pro-Thr-Asn- Val-Gly-Ser-Lys-Ala-Phe- NH2 Ser-Asp-Thr-Cys-Trp-Ser-Thr-Thr-Ser-Phe-Gln-Lys- Lys-Thr-Ile- His-Cys-Lys-Trp-Arg-Glu-Lys-Pro-Leu- Met-Leu-Met | |
| MGOP 27 | | SEQ ID NO:46 |
| Sarafotoxin S6a (cardiotoxin isotoxin) | | SEQ ID NO:32 |
| Sarafotoxin S6b (potent coronary constrictor activity) | Cys-Ser-Cys-Lys-Asp-Met-Thr-Asp-Lys-Glu-Cys-Leu- Asn-Phe-Cys- His-Gln-Asp-Val-Ile-Trp Cys-Ser-Cys-Lys-Asp-Met-Thr-Asp-Lys-Glu-Cys-Leu- Tyr-Phe-Cys- His-Gln-Asp-Val-Ile-Trp | SEQ ID NO:33 |
| Sarafotoxin S6c (cause strong vasoconstriction of coronary vessels) | Cys-Thr-Cys-Asn-Asp-Met-Thr-Asp-Glu-Glu-Cys-Leu- Asn-Phe-Cys- His-Gln-Asp-Val-Ile-Trp | SEQ ID NO:34 |
| Septide (elective Substance Preceptor Peptide) | | SEQ ID NO:47 |
| Somatostatin-28 | pGlu-Phe-Phe-Pro-Leu-Met-NH2 Ser-Ala-Asn-Ser-Asn-Pro-Ala-Met-Ala-Pro-Arg-Glu- Arg-Lys-Ala- Gly-Cys-Lys-Asn-Phe-Phe-Trp-Lys-Thr- Phe-Thr-Ser-Cys | SEQ ID NO:48 |
| Endothelin-1 (Human, Bovine, Canine, Mouse, Porcine, Rat) | | SEQ ID NO:49 |
| Growth Hormone Releasing Factor (Human) | Cys-Ser-Cys-Ser-Ser-Leu-Met-Asp-Lys-Glu-Cys-Val- Tyr-Phe-Cys- His-Leu-Asp-Ile-Ile-Trp Tyr-Ala-Asp-Ala-Ile-Phe-Thr-Asn-Ser-Tyr-Arg-Lys-Val- Leu-Gly- Gln-Leu-Ser-Ala-Arg-Lys-Leu-Leu-Gln-Asp- Ile-Met-Ser-Arg-Gln- Gln-Gly-Glu-Ser-Asn-Gln-Glu- Arg-Gly-Ala-Arg-Ala-Arg-Leu-NH2  | SEQ ID NO:50 |
| amylin amide | | SEQ ID NO:51 |

The neurogenesis modulating agents (also referred to as the agents) of this disclosure arc as listed in this section. It is understood that these neurogenesis modulating agent (agents) may be used wherever neurogenesis modulating agent or agents is specified in this specification. The neurogenesis modulating agent of the invention is selected from the group of Thyrocalcitonin, Calcitonin, Glucagon-Like Peptide-1 (7-37), Exendin-3 and Exendin-4, and analogs thereof, and a combination thereof, or a cAMP analog, wherein said cAMP analog is selected from the group consisting of 8-pCPT-2-O-Me-cAMP, 8-Br-cAMP, Rp-cAMPS, 8-Cl-cAMP, Dibutyryl cAMP, pCPT-cAMP, and N6-monobutyryladenosine 3',5'-cyclic monophosphate. In a preferred embodiment of the invention, the neurogenesis modulating agent increases or maintains the amount of doublecortin positive cells or the percentage of doublecortin positive cells in a cell population or neural tissue.

### 2. Production of Neurogenesis modulating agents

Neurogenesis modulating agents may be produced using known techniques of chemical synthesis including the use of peptide synthesizers.

In some embodiments of the invention, the neurogenesis modulating agent is a peptide or protein. Peptides and proteins may be synthesized chemically using commercially available peptide synthesizers. Chemical synthesis of peptides and proteins can be used for the incorporation of modified or unnatural amino acids, including D-amino acids and other small organic molecules. Replacement of one or more L-amino acids in a peptide or protein with the corresponding D-amino acid isoforms can be used to increase resistance to enzymatic hydrolysis, and to enhance one or more properties of biological activity, i.e., receptor binding, functional potency, or duration of action. See, e.g., Doherty, et al., 1993. J. Med. Chem. 36: 2585-2594; Kirby, et al., 1993, J. Med. Chem. 36:3802-3808; Morita, et al., 1994, FEBS Lett. 353: 84-88; Wang, et al., 1993 Int. J. Pept. Protein Res. 42: 392-399; Fauchere and Thiunieau, 1992. Adv. Drug Res. 23: 127-159.

Introduction of covalent cross-links into a peptide or protein sequence can conformationally and topographically constrain the peptide backbone. This strategy can be used to develop peptide or protein analogs of neurogenesis modulating agents with increased potency, selectivity, and stability. A number of other methods have been used successfully to introduce conformational constraints into amino acid sequences in order to improve their potency, receptor selectivity, and biological half-life. These include the use of (i) C_{α}-methylamino acids (see, e.g., Rose, et al., Adv. Protein Chem. 37; 1-109 (1985); Prasad and Balaram, CRC Crit. Rev. Biochem., 16: 307-348 (1984)); (ii) N_{α}-methylamino acids (see, e.g., Aubry, et al., Int. J. Pept. Protein Res., 18: 195-202 (1981); Manavalan and Momany, Biopolymers, 19: 1943-1973 (1980)); and (iii) α,β-unsaturated amino acids (see, e.g., Bach and Gierasch, Biopolymers, 25: 5175-S192 (1986); Singh, et al., Biopolymers, 26; 819-829 (1987)). These and many other amino acid analogs are commercially available, or can be easily prepared. Additionally, replacement of the C- terminal acid with an amide can be used to enhance the solubility and clearance of a peptide or protein.

Alternatively, a neurogenesis modulating agent may be obtained by methods well known in the art for recombinant peptide or protein expression and purification. A DNA molecule encoding the neurogenesis modulating agent can be generated. The DNA sequence is known or can be deduced from the amino acid sequence based on known codon usage. See, e.g., Old and Primrose, Principles of Gene Manipulation 3rd ed., Blackwell Scientific Publications, 1985; Wada et al., Nucleic Acids Res. 20: 2111-2118(1992). Preferably, the DNA molecule includes additional sequence, e.g., recognition sites for restriction enzymes which facilitate its cloning into a suitable cloning vector, such as a plasmid. Nucleic acids may be DNA, RNA, or a combination thereof. Nucleic acids encoding the neurogenesis modulating agent may be obtained by any method known within the art (e.g., by PCR amplification using synthetic primers hybridizable to the 3'- and 5'-termini of the sequence and/or by cloning from a cDNA or genomic library using an oligonucleotide sequence specific for the given gene sequence, or the like). Nucleic acids can also be generated by chemical synthesis.

Any of the methodologies known within the relevant art regarding the insertion of nucleic acid fragments into a vector may be used to construct expression vectors that contain a chimeric gene comprised of the appropriate transcriptional/translational control signals and neurogenesis modulating agent-coding sequences. Promoter/enhancer sequences within expression vectors may use plant, animal, insect, or fungus regulatory sequences, as provided herein. A host cell can be any prokaryotic or eukaryotic cell. For example, the peptide can be expressed in bacterial cells such as E. coli, yeast, insect cells, fungi or mammalian cells. Other suitable host cells are known to those skilled in the art. In one embodiment, a nucleic acid encoding a neurogenesis modulating agent is expressed in mammalian cells using a mammalian expression vector.

Exemplary bacterial vectors include, but are not limited to: pUC plasmids such as pUC7, pUC8, pUC9, pUC12, pUC13, pUC18, pUC19, pUC118, pUC119; pBR plasmids such as pBR322, pBR325 (Biorad Laboratories, Richmond, CA); pSPORT 1; pT7/T3a-18, pT7/T3a-19; pGEM plasmids such as pGEM3Z, pGEM-4Z, pGEM-3Zf(+/-), pGEM-5Zf(+/-), pGEM-7Zf(+/-), pGEM-9Zf(+/-), PGEM-11Zf(+/-), PGEM-13Zf(+) (Promega, Madison, WI); pSP plasmids such as pSP70, pSP71, pSP72, pSP73, pSP64, pSP65, pSP64 poly(A), pAlter-1; BLUSCRIPT plasmids such as pBS II SK(+/-), pBS II KS(+/-), pCR-Script SK(+/-), pBS(+/-) pT7-7, pBS-KS(+/-) pT7-7A, pBS-SK(+/-) pTZ18R; pTZ1 8U; pTZ19R, pT7-1 pTZ19U; pT7-2, and pQE50 (Qiagen, Chatsworth, CA). Exemplary bacterial host cells include, but are not limited to: BMH 71-18 mut S, C600, C600 hfl, DH1, DH5 α, DH5 αF', DMI, HB101, JM83, JM101, JM103, JM105, JM107, JM108, JM109, JM109(DE3), LE392, KW251, MM294, NM522, NM538, NM539, RR1, Y1088, Y1089, Y1090, AG1, JM110, K802, SCS1, SCS110, XL-1 Blue, XL1-Blue MRF', and XLR1-Blue MR. Many strains are commercially available (see, e.g., ATCC, Rockville, MD; GIBCO BRL, Gaithersburg, MD).

Examples of mammalian vectors include: pCDM8 (Seed (1987) Nature 329:840; Invitrogen) and pMT2PC (Kaufman et al. (1987) EMBO J. 6: 187-195), pCMVβ (Invitrogen), pcDNA3 (Invitrogen), pET-3d (Novagen), pProEx-1 (Life Technologies), pFastBac 1 (Life Technologies), pSFV (Life Technologies), pcDNA3, pcDNA.4, and pcDNA6 (Invitrogen), pSL301 (Invitrogen), pSE280 (Invitrogen), pSE380 (Invitrogen), pSE420 (Invitrogen), pTrcHis A,B,C (Invitrogen), pRSET A,B,C (Invitrogen), pYES2 (Invitrogen), pAC360 (Invitrogen), pVL1392 and pV11392 (Invatrogen), pZeoSV (Invitrogen), pRc/CMV (Invitrogen), pRc/RSV (Invitrogen), pREP4 (Invitrogen), pREP7 (Invitrogen), pREP8 (Invitrogen), pREP9 (Invitrogen), pREP10 (Invitrogen), pCEP4 (Invitrogen), pEBVHis (Invitrogen), and lambda-Pop6. Examples of eukaryotic host cells that can be used to express a fusion protein of the invention include Chinese hamster ovary (CHO) cells (e.g., ATCC Accession No. CCL-61), NIH Swiss mouse embryo cells NIH/3T3 (e.g., ATCC Accession No. CRL-1658), and Madin-Darby bovine kidney (MDBK) cells (ATCC Accession No. CCL-22). Other vectors and host cells would be apparent to one skilled in the art.

The host cells can be used to produce (i.e., overexpress) peptide in culture, for example by culturing the host cell (into which a recombinant expression vector encoding the peptide or protein has been introduced) in a suitable medium such that peptide is produced. The method further involves isolating peptide or protein from the medium or the host cell. Ausubel et al., (Eds). In: Current Protocols in Molecular Biology. J. Wiley and Sons, New York, NY. 1998.

The biologically expressed neurogenesis modulating agent may be purified using known purification techniques. An "isolated" or "purified" recombinant peptide or protein, or biologically active portion thereof, means that said peptide or protein is substantially free of cellular material or other contaminating proteins from the cell or tissue source from which it is derived. The language "substantially free of cellular material" includes preparations in which the peptide or protein is separated from cellular components of the cells from which it is isolated or recombinantly produced. In one embodiment, the language "substantially free of cellular material" includes preparations of peptide or protein having less than about 30% (by dry weight) of product other than the desired peptide or protein (also referred to herein as a "contaminating protein"), more preferably less than about 20% of contaminating protein, still more preferably less than about 10% of contaminating protein, and most preferably less than about 5% contaminating protein. When the peptide or protein, or biologically active portion thereof, is recombinantly produced, it is also preferably substantially free of culture medium, i.e., culture medium represents less than about 20%, more preferably less than about 10%, and most preferably less than about 5% of the volume of the peptide or protein preparation.

Preferably, the analog, of the neurogenesis modulating agent is functionally active. As utilized herein, the term "functionally active" refers to species displaying one or more known functional attributes of neurogenesis.

Analogs of a neurogenesis modulating agent of the invention or individual moieties can be produced by various methods known within the art. For example, the amino acid sequences may be modified by any number of methods known within the art. See e.g., Sambrook, et al., 1990. Molecular Cloning: A Laboratory Manual, 2nd ed., (Cold Spring Harbor Laboratory Press; Cold Spring Harbor, NY). Modifications include: glycosylation, acetylation, phosphorylation, amidation, derivatization by known protecting/blocking groups, linkage to an antibody molecule or other cellular reagent. Any of the numerous chemical modification methodologies known within the art may be utilized including specific chemical cleavage by cyanogen bromide, trypsin, chymotrypsin, papain, V8 protease, NaBH₄, acetylation, formylation, oxidation, reduction and metabolic synthesis in the presence of tunicamycin.

Analogs may be full length or other than full length, if said analog contains a modified nucleic acid or amino acid, as described infra. Analogs of the neurogenesis modulating agent include molecules comprising regions that are substantially homologous in various embodiments, of at least 30%, 40%, 50%, 60%, 70%, 80%, 90% or preferably 95% amino acid identity when: (i) compared to an amino acid sequence of identical size; (ii) compared to an aligned sequence in that the alignment is done by a computer homology program known within the art (e.g., Wisconsin GCG software) or (iii) the encoding nucleic acid is capable of hybridizing to a sequence encoding the aforementioned peptides under stringent (preferred), moderately stringent, or non-stringent conditions. See, e.g., Ausubel, et al., Current Protocols in Molecular Biology, John Wiley and Sons, New York, NY, 1993.

### 3. Fusion Proteins Comprisine Neurogenesis modulating agents

In various aspects of the invention, the protein and peptide neurogenesis modulating agents disclosed herein can be expressed as fusion proteins. As non-limiting examples, the fusion proteins can include one or more poly-His tag, c-myc tag, E-tag, S-tag, FLAG-tag, Glu-Glu tag, HA tag, HSV-tag, V5, VSV-g, β-galalactosidase, GFP, GST, luciferase, maltose binding protein, alkaline phosphatase cellulose binding domain, Fc domain, or other heterologous sequences. One of ordinary skill in the art can prepare such fusion proteins using well known molecular biology techniques. For example, conventional recombinant DNA methodologies can be used to generate fusion proteins useful in the practice of the invention.

According to such methods, fusion constructs can be generated, and the resulting DNAs can be integrated into expression vectors, and expressed to produce the fusion proteins for use in the invention. An appropriate host cell can be transformed or transfected with the expression vector, and utilized for the expression and/or secretion of the target protein. Currently preferred host cells for use in the invention include immortal hybridoma cells, NS/O myeloma cells, 293 cells, Chinese hamster ovary cells, HELA cells, and COS cells. Prokaryotic host cells can also be modified to comprise vectors for expressing fusion proteins, such as pGEX (Pharmacia Biotech Inc; Smith, D. B. and Johnson, K. S. (1988) Gene 67:31-40), pMAL (New England Biolabs, Beverly, MA), and pRIT5 (Pharmacia, Piscataway, NJ).

For some purposes, it may be desireable to include a signal sequence in a fusion protein for use in the invention. Signal sequences that may be used with the expression constructs for use in the invention include antibody light chain signal sequences, e.g., antibody 14.18 (Gillies et, al. (1989) J. Immunol. Meth. 125:191), antibody heavy chain signal sequences, e.g., the MOPC141 antibody heavy chain signal sequence (Sakano et al. (1980) Nature 286:5774), and any other signal sequences which are known in the art (see, e.g., Watson (1984) Nucleic Acids Research 12:5145). A detailed discussion of signal peptide sequences is provided by von Heijne (1986) Nucleic Acids Research 14:4683.

As would be apparent to one of skill in the art, the suitability of a particular signal sequence for use in a vector for secretion may require some routine experimentation. Such experimentation will include determining the ability of the signal sequence to direct the secretion of a fusion protein and also a determination of the optimal configuration, genomic or cDNA, of the sequence to be used in order to achieve efficient secretion of fusion proteins. Additionally, one skilled in the art is capable of creating a synthetic signal peptide following the rules presented by von Heijne, referenced above, and testing for the efficacy of such a synthetic signal sequence by routine experimentation.

A fusion protein for use in the invention can include a proteolytic cleavage site to provide for the proteolytic cleavage of the encoded fusion protein. In this way, the heterologous domain (e.g., GST protein) can be separated from the peptide or protein sequence of interest. Useful proteolytic cleavage sites include amino acids sequences that are recognized by proteolytic enzymes such as trypsin, plasmin, or enterokinase K. Many cleavage site/cleavage agent pairs are known (see, for example, U.S. Pat. No. 5,726,044).

After synthesis by chemical or biological methods, The peptides and proteins for use in the invention can be purified so that they are substantially free of chemical precursors or other chemicals using standard purification techniques. The language "substantially free of chemical precursors or other chemicals" includes preparations in which the peptide or protein is separated from chemical precursors or other chemicals that are involved in synthesis. In one embodiment, the language "substantially free of chemical precursors or other chemicals" includes preparations having less than about 30% (by dry weight) of chemical precursors or other chemicals, more preferably less than about 20% chemical precursors or other chemicals, still more preferably less than about 10% chemical precursors or other chemicals, and most preferably less than about 5% chemical precursors or other chemicals.

### 4. Compositions Comprising Neurogenesis modulating agent(s)

Also described herein are pharmaceutical compositions comprising a neurogenesis modulating agent of the invention, The neurogenesis modulating agents of the invention can be formulated into pharmaceutical compositions that can be used as therapeutic agents for the treatment of a neurological diseases (disorders). These compositions are discussed in this section. It is understood that any pharmaceutical compositions and chemicals discussed in this section can be a component of a pharmaceutical composition comprising one or more neurogenesis modulating agents.

Neurogenesis modulating agents and co-administered agents can be incorporated into pharmaceutical compositions suitable for administration. Such compositions typically comprise the agent and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions. Modifications can be made to the agents to affect solubility or clearance of the peptide. Peptidic molecules may also be synthesized with D-amino acids to increase resistance to enzymatic degradation. In some cases, the composition can be co-administered with one or more solubilizing agents, preservatives, and permeation enhancing agents.

Preferably, the pharmaceutical composition is used to treat diseases by stimulating neurogenesis (i.e., cell growth, proliferation, migration, survival and/or differentiation). For treatment, a use of the invention comprises administering to the subject an effective amount of a pharmaceutical composition including an agent of the invention (1) alone in a dosage range of 0.001 ng/kg/day to 500 ng/kg/day, preferably in a dosage range of 0.05 to 200 ng/kg/day, (2) in a combination permeability increasing factor, or (3) in combination with a locally or systemically co-administered agent.

A pharmaceutical composition for use in the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile, physiologically acceptable diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Oral administration refers to the administration of the formulation via the mouth through ingestion, or via any other part of the gastrointestinal system including the esophagus or through suppository administration. Parenteral administration refers to the delivery of a composition, such as a composition comprising a neurogenesis modulating agent by a route other than through the gastrointestinal tract (e.g., oral delivery). In particular, parenteral administration may be via intravenous, subcutaneous, intramuscular or intramedullary (i.e., intrathecal) injection. Topical administration refers to the application of a pharmaceutical agent to the external surface of the skin or the mucous membranes (including the surface membranes of the nose, lungs and mouth), such that the agent crosses the external surface of the skin or mucous membrane and enters the underlying tissues. Topical administration of a pharmaceutical agent can result in a limited distribution of the agent to the skin and surrounding tissues or, when the agent is removed from the treatment area by the bloodstream, can result in systemic distribution of the agent.

In a preferred form of topical administration, the neurogenesis promoting agent is delivered by transdermal delivery. Transdermal delivery refers to the diffusion of an agent across the barrier of the skin, The skin (stratum corneum and epidermis) acts as a barrier and few pharmaceutical agents are able to penetrate intact skin. In contrast, the dermis is permeable to many solutes and absorption of drugs therefor occurs more readily through skin that is abraded or otherwise stripped of the epidermis to expose the dermis. Absorption through intact skin can be enhanced by placing the active agent in an oily vehicle before application to the skin (a process known as inunction). Passive topical administration may consist of applying the active agent directly to the treatment site in combination with emollients or penetration enhancers, Another method of enhancing delivery through the skin is to increase the dosage of the pharmaceutical agent. The dosage for topical administration may be increased up to ten, a hundred or a thousand folds more than the usual dosages stated elsewhere in this disclosure.

Pharmaceutical compositions suitable for injectable use include sterile aqueous solutions (where water soluble) or dispersions and sterile powders for the extemporaneous preparation of sterile injectable solutions or dispersion. For intravenous administration, physiologically acceptable, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Physiologically acceptable carriers maybe any carrier known in the field as suitable for pharmaceutical (i.e., topical, oral, and parenteral) application. Suitable pharmaceutical carriers and formulations are described, for example, in Remington's Pharmaceutical Sciences (19th ed.) (Genarro, ed. (1995) Mack Publishing Co., Easton, Pa.). Preferably, pharmaceutical carriers are chosen based upon the intended mode of administration of the neurogenesis modulating agent. The pharmaceutically acceptable carrier may include, for example, emollients, humectants, thickeners, silicones, and water. Suitable formulations that include pharmaceutically acceptable excipients for introducing the neurogenesis modulating agent to the bloodstream by other than injection routes can be found in Remington's Pharmaceutical Sciences (19th ed.) (Genarro, ed. (1995) Mack Publishing Co., Easton, Pa.).

Specific examples of carriers include hydrocarbon oils and waxes such as mineral oil, petrolatum, paraffin, ceresin, ozokerite, microcrystalline wax, polyethylene, and perhydrosqualene; triglyceride such as vegetable oil, animal fats, castor oil, cocoa butter, safflower oil, cottonseed oil, corn oil, olive oil, cod liver oil, almond oil, avocado oil, palm oil, sesame oil, squalene, and maleated soybean oil; acetoglycerides, such as acetylated monoglycerides; ethoxylated glycerides, such as ethoxylated glyceryl monostearate; alkyl esters of fatty acids such as methyl, isopropyl, and butyl, hexyl laurate, isohexyl laurate, isohexyl palmitate, isopropyl palmitate, decyl oleate, isodecyl oleate, hexadecyl stearate, decyl stearate, isopropyl isostearate, diisopropyl adipate, diisohexyl adipate, dihexyldccyl adipate, diisopropyl sebacate, lauryl lactate, myristyl lactase, and cetyl lactate esters of fatty acid; alkenyl esters of fatty acids such as oleyl myristate, oleyl stearate, and oleyl oleate; fatty acids such as pelargonic, lauric, myristic, palmitic, stearic, isostearic, hydroxystearic, oleic, linoleic, ricinoleic, arachidic, behenic, and erucic acids; fatty alcohols such as lauryl, myristyl, cetyl, hexadecyl, stearyl, isostearyl, hydroxystearyl, oleyl, ricinoleyl, behenyl, erucyl, and 2-octyl dodecanyl alcohols; fatty alcohol ethers such as lauryl, cetyl, stearyl, isostearyl, oleyl, and cholesterol alcohols, having attached thereto from I to 50 ethylene oxide groups or 1 to 50 propylene oxide groups; ether-esters such as fatty acid esters of ethoxylated fatty alcohols.

Also included are lanolin and derivatives such as lanolin, lanolin oil, lanolin wax, lanolin alcohols, lanolin fatty acids, isopropyl lanolate, ethoxylated lanolin, ethoxylated lanolin alcohols, ethoxylated cholesterol, propoxylated lanolin alcohols, acetylated lanolin alcohols, lanolin alcohols linoleate, lanolin alcohols ricinoleate, acetate of lanolin alcohols ricinoleate, acetate of ethoxylated alcohols-esters, hydrogenolysis of lanolin, ethoxylated hydrogenated lanolin, ethoxylated sorbitol lanolin, and liquid and semisolid lanolin absorption bases; polyhydric alcohol esters such as ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 mono- oleate, polypropylene glycol 2000 monostearate, ethoxylated propylene glycol monostearatc, glyceryl mono- and di-fatty acid esters, polyglyccrol poly-fatty esters, ethoxylated glyceryl monostearate, 1,3-butylene glycol monostearate, 1,3-butylene glycol distearate, polyoxyethylene polyol fatty acid esters, sorbitan fatty acid esters, and polyoxyethylene sorbitan fatty acid esters are satisfactory polyhydric alcohol esters.

Further included are waxes such as beeswax, spermaceti, myristyl myristate, stearyl stearatepolyoxyethylene sorbitol beeswax, carnauba and candelilla waxes; phospholipids such as lecithin and derivatives; sterols such as cholesterol and cholesterol fatty acid esters, amides such as fatty acid amides, ethoxylated fatty acid amides, and solid fatty acid alkanolamides. In addition, the neurogenesis modulating agent and the pharmaceutically acceptable carrier may be enclosed in a hard or soft shell gelatin capsule, compressed into tablets, or incorporated directly into the individual's diet. Specifically, the neurogenesis modulating agent may be incorporated with excipients and used in the form of ingestible tablets, buccal tablets, troches, capsules, elixirs, suspensions, syrups, wafers, and the like. When the neurogenesis modulating agent is administered orally, it may be mixed with other food forms and pharmaceutically acceptable flavour enhancers. When the neurogenesis modulating agent is administered enterally, they may be introduced in a solid, semi-solid, suspension, or emulsion form and may be compounded with any number of well-known, pharmaceutically acceptable additives. Sustained release oral delivery systems and/or enteric coatings for orally administered dosage forms are known in the art and also contemplated.

Oral compositions generally include a physiologically acceptable, inert diluent or an edible carrier. They can be enclosed in gelatin capsules or compressed into tablets. For the purpose of oral therapeutic administration, the neurogenesis modulating agent of the invention can be incorporated with physiological excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; physiologically acceptable excipients such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

Where a neurogenesis modulating agent for use in the invention is administered as a topical agent, the composition for use in the invention may optionally comprise other agents known to have a cosmetic or beneficial effect on the skin. Such agents include, for example, antioxidants, sunscreens, a pH buffer, and a combination thereof While any antioxidant that is chemically compatible may be used, preferred antioxidants include amino acids such as glycine, histidine, tyrosine, and tryptophan; imidazoles such as urocanic acid; peptides such as D,L-carnosine, D-carnosine, L-carnosine and anserine; carotenoids; carotenes such as alpha-carotene, beta-carotene, and lycopene; lipoic acid such as dihydrolipoic acid; thiols such as aurothioglucose, propylthiouracil, thioredoxin, glutathione, cysteine, cystine, and cystamine; dilauryl thiodipropionate; distearyl thiodipropionate; thiodipropionic acid; sulphoximine compounds such as buthionine-sulphoximines, homocysteine-sulphoximine, buthionine-sulphones, penta-, hexa- and heptathionine-sulphoximine; metal chelating agents such as alpha-hydroxy-fatty acids, palmitic acid, phytic acid, lactoferrin EDTA and EGTA; alpha-hydroxy acids such as citric acid, lactic acid, and malic acid; unsaturated fatty acids such as gamma-linolenic acid, linoleic acid and oleic acid; folio acid; ubiquinone and ubiquinol.

Sterile injectable solutions can be prepared by incorporating the neurogenesis modulating agent for use in the invention (e.g., a nucleic acid, peptide, fusion protein, antibody, affibody, and the like) in the required amount in an appropriate solvent with one or a combination of ingredients enumerated above, as required, followed by filtered sterilization. Generally, dispersions are prepared by incorporating the neurogenesis modulating agent into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

A number of systems that alter the delivery of injectable drugs can be used to change the pharmacodynamic and pharmacokinetic properties of therapeutic agents (see, e.g., K. Reddy, 2000, Annals of Pharmacotherapy 34:915-923). Drug delivery can be modified through a change in formulation (e.g., continuous-release products, liposomes) or an addition to the drug molecule (e.g., pegylation), Potential advantages of these drug delivery mechanisms include an increased or prolonged duration of pharmacologic activity, a decrease in adverse effects, and increased patient compliance and quality of life. Injectable continuous-release systems deliver drugs in a controlled, predetermined fashion and are particularly appropriate when it is important to avoid large fluctuations in plasma drug concentrations. Encapsulating a drug within a liposome can produce a prolonged half-life and an increased distribution to tissues with increased capillary permeability (e.g., tumors). Pegylation provides a method for modification of therapeutic peptides or proteins to minimize possible limitations (e.g., stability, half-life, immunogenicity) associated with these neurogenesis modulating agents.

In accordance with the invention, one or more neurogenesis modulating agents can be formulated with lipids or lipid vehicles (e.g., micells, liposomes, microspheres, protocells, protobionts, liposomes, coacervates, and the like) to allow formation of multimers. Similarly, neurogenesis modulating agents can be multimerized using pegylation, cross-linking, disulfide bond formation, formation of covalent cross-links, glycosylphosphatidylinositol (GPI) anchor formation, or other established methods. The multimerized neurogenesis modulating agent can be formulated into a pharmaceutical composition, and used to increase or enhance their effects.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For administration by inhalation, the neurogenesis modulating agents for use in the invention can be delivered in the form of an aerosol spray from pressured container or dispenser that contains a suitable propellant, e.g., a gas such as carbon dioxide, or a nebulizer. For transdermal administration, the neurogenesis modulating agents for use in the invention can be formulated into ointments, salves, gels, or creams as generally known in the art. The neurogenesis modulating agents can also be prepared in the form of suppositories (e.g., with conventional suppository bases such as cocoa butter and other glycerides) or retention enemas for rectal delivery.

In one embodiment, the neurogenesis modulating agents for use in the invention are prepared with carriers that will protect the neurogenesis modulating agent against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

It is especially advantageous to formulate oral or parenteral compositions in dosage unit form for case of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms for use in the invention are dictated by and directly dependent on the unique characteristics of the active compound and the particular therapeutic effect to be achieved, and the limitations inherent in the art of compounding such an active compound for the treatment of individuals.

In other embodiments, the neurogenesis modulating agent is administered in a composition comprising at least 90% pure neurogenesis modulating agent. Preferably the neurogenesis modulating agent is formulated in a medium providing maximum stability and the least formulation-related side effects. In addition to the neurogenesis modulating agent, the composition for use in the invention will typically include one or more protein carrier, buffer, isotonic salt, and stabilizer.

Compositions that include one or more neurogenesis modulating agents of the invention can be administered in any conventional form, including in any form known in the art in which it may either pass through or by-pass the blood-brain barrier. Methods for allowing factors to pass through the blood-brain barrier include minimizing the size of the factor, providing hydrophobic factors which may pass through more easily, conjugating the protein neurogenesis modulating agent or other agent to a carrier molecule that has a substantial permeability coefficient across the blood brain barrier (see, e.g., U.S. Patent 5,670,477).

In some instances, the neurogenesis modulating agent can be administered by a surgical procedure implanting a catheter coupled to a pump device. The pump device can also be implanted or be extracorporally positioned. Administration of the neurogenesis modulating agent can be in intermittent pulses or as a continuous infusion. Devices for injection to discrete areas of the brain are known in the art (see, e.g., U.S. Patent Nos. 6,042,579; 5,832,932; and 4,692,147).

### 5. Method for Reducing a Symptom of a Disorder by Administering Neurogenesis modulating agent(s)

One embodiment of the invention is directed to reducing a symptom of a disorder in a patient by administering a neurogenesis modulating agent of the invention to the patient. In that use, one or more neurogenesis modulating agent is directly administered to the animal which will induce additional proliferation and/or differentiation of an adult neural tissue of said animal. Such in *vivo* uses allows disorders caused by cells lost, due to injury or disease, to be endogenously replaced. This will obviate the need for transplanting foreign cells into a patient

A neurogenesis modulating agent for use in the invention can be administered systemically to a patient. In a preferred embodiment, the neurogenesis modulating agent is administered locally to any loci implicated in the CNS disorder pathology, i.e. any loci deficient in neural cells as a cause of the disease. For example, the neurogenesis modulating agent can be administered locally to the ventricle of the brain, substantia nigra, striatum, locus ceruleous, nucleus basalis Meynert, pedunculopontine nucleus, cerebral cortex, and spinal cord. A central nervous system disorder of the present invention is selected from neurodegenerative disorders, ischemic disorders, neurological traumas, and learning and memory disorders.

Administration of growth factors can be done by any method, including injection cannula, transfection of cells with growth hormone-expressing vectors, injection, timed-release apparatus which can administer substances at the desired site, and the like. Pharmaceutical compositions can be administered by any method, including injection cannula, injection, oral administration, timed-release apparati and the like. Any growth factor can be used, particularly EGF, TGF.alpha., FGF-1, FGF-2 and NGF. Growth factors can be administered in any manner known in the art in which the factors may either pass through or by-pass the blood-brain barrier. Methods for allowing factors to pass through the blood-brain barrier include minimizing the size of the factor, or providing hydrophobic factors which may pass through more easily.

The use of the invention takes advantage of the fact that stem cells are located in the tissues lining ventricles of mature brains offers. Neurogenesis may be induced by administering a neurogenesis modulating agent of the invention directly to these sites and thus avoiding unnecessary systemic administration and possible side effects. It may be desirable to implant a device that administers the composition to the ventricle and thus, to the neural stem cells. For example, a cannula attached to an osmotic pump may be used to deliver the composition. Alternatively, the composition may be injected directly into the ventricles. The cells can migrate into regions that have been damaged as a result of injury or disease. Furthermore, the close proximity of the ventricles to many brain regions would allow for the diffusion of a secreted neurological agent by the cells (e.g., stem cells or their progeny).

The invention provides agents for use in inducing neurogenesis *in vivo* or *in vitro,* which can be used to treat various diseases and disorders of the CNS as described in detail herein. The term "treating" in its various grammatical forms in relation to the present invention refers to preventing, curing, reversing, attenuating, alleviating, ameliorating minimizing, suppressing, or halting the deleterious effects of a neurological disorder, disorder progression, disorder causative agent (e.g., bacteria or viruses), injury, trauma, or other abnormal condition. Symptoms of neurological disorders include, tension, abnormal movements, abnormal behavior, tics, hyperactivity, combativeness, hostility, negativism, memory defects, sensory defects, cognitive defects, hallucinations, acute delusions, poor self-care, and sometimes withdrawal and seclusion.

Abnormal movement symptoms include a wide variety of symptoms that can range from unconscious movements that interfere very little with quality of life, to quite severe and disabling movements. Examples of symptoms which are seen associated with neurological disorders include involuntary tongue protrusions, snake-like tongue movements, repetitive toe and finger movements, tremors of extremities or whole body sections, tics, muscular rigidity, slowness of movement, facial spasms, acute contractions of various muscles, particularly of the neck and shoulder which may eventually lead to painful, prolonged muscle contraction, restlessness, distress and an inability to remain still. Abnormal behavioral symptoms, some of which are motor in nature, include irritability, poor impulse control, distractibility, aggressiveness, and stereotypical behaviors that are commonly seen with mental impairment such as rocking, jumping, running, spinning, flaying.

Any of the uses of the invention may be used to alleviate a symptom of a neurological disease or disorder such as Parkinson's disease (shaking palsy), including primary Parkinson's disease, secondary parkinsonism, and postencephalitic parkinsonism; drug-induced movement disorders, including parkinsonism, acute dystonia, tardive dyskinesia, and neuroleptic malignant syndrome; Huntington's disease (Huntington's chorea; chronic progressive chorea; hereditary chorea); delirium (acute confusional state); dementia; Alzheimer's disease; non-Alzheimer's dementias, including Lewy body dementia, vascular dementia, Binswanger's dementia (subcortical arteriosclerotic encephalopathy), dementia pugilistica, normal-pressure hydrocephalus, general paresis, frontotemporal dementia, multi-infaret dementia, and AIDS dementia; age-associated memory impairment (AAMI); amnesias, such as retrograde, anterograde, global, modality specific, transient, stable, and progressive amnesias, and posttraumatic amnesias, and Korsakoffs disease.

Other diseases and disorders include idiopathic orthostatic hypotension, Shy-Drager syndrome, progressive supranuclear palsy (Steele-Richardson-Olszewski syndrome); structural lesions of the cerebellum, such as those associated with infarcts, hemorrhages, or tumors; spinocerebellar degenerations such as those associated with Friedreich's ataxia, abetalipoproteinemia (e.g., Bassen-Kornzweig syndrome, vitamin E deficiency), Refsum's disease (phytanic acid storage disease), cerebellar ataxias, multiple systems atrophy (olivopontocerebellar atrophy), ataxia-telangiectasia, and mitochondrial multisystem disorders; acute disseminated encephalomyelitis (postinfectious encephalomyelitis); adrenoleukodystrophy and adrenomyeloneuropathy; Leber's hereditary optic atrophy; HTLV-associated myelopathy; and multiple sclerosis; motor neuron disorders such as amyotrophic lateral sclerosis, progressive bulbar palsy, progressive muscular atrophy, primary lateral sclerosis and progressive pseudobulbar palsy, and spinal muscular atrophies such as type I spinal muscular atrophy (Werdnig-Hoffmann disease), type II (intermediate) spinal muscular atrophy, type III spinal muscular atrophy (Wohlfart-Kugelberg-Welander disease), and type IV spinal muscular atrophy.

Further diseases and disorders include plexus disorders such as plexopathy and acute brachial neuritis (neuralgic amyotrophy); peripheral neuropathies such as mononeuropathies, multiple mononeuropathies, and polyneuropathies, including ulnar nerve palsy, carpal tunnel syndrome, peroneal nerve palsy, radial nerve palsy, Guillain-Barré syndrome (Landry's ascending paralysis; acute inflammatory demyelinating polyradiculoneuropathy), chronic relapsing polyneuropathy, hereditary motor and sensory neuropathy, e.g., types I and II (Charcot-Marie-Tooth disease, peroneal muscular atrophy), and type III (hypertrophic interstitial neuropathy, Dejerine-Sottas disease); disorders of neuromuscular transmission, such as myasthenia gravis; neuro-ophthalmologic disorders such as Horner's syndrome, internuclear ophthalmoplegia, gaze palsies, and Parinaud's syndrome; cranial nerve palsies, trigeminal neuralgia (Tic Douloureux); Bell's palsy; and glossopharyngeal neuralgia; radiation-induced injury of the nervous system; chemotherapy-induced neuropathy (e.g., encephalopathy); taxol neuropathy; vincristine neuropathy; diabetic neuropathy; autonomic neuropathies; polyneuropathie;, and mononeuropathies; and ischemic syndromes such as transient ischemic attacks, subclavian steal syndrome, drop attacks, ischemic stroke, hemorrhagic stroke, and brain infarction.

For treatment of Huntington's Disease, Alzheimer's Disease, Parkinson's Disease, and other neurological disorders affecting primarily the forebrain, one or more of the disclosed neurogenesis modulating agents, with or without growth factors or other neurological agents would be delivered to the ventricles of the forebrain to affect *in vivo* modification or manipulation of the cells. For example, Parkinson's Disease is the result of low levels of dopamine in the brain, particularly the striatum. It would be advantageous to induce a patient's own quiescent stem cells to begin to divide *in vivo,* thus locally raising the levels of dopamine. The uses and compositions for use in the present invention provide an alternative to the use of drugs and the controversial use of large quantities of embryonic tissue for treatment of Parkinson's disease. Dopamine cells can be generated in the striatum by the administration of a composition comprising growth factors to the lateral ventricle.

For the treatment of MS and other demyelinating or hypomyelinating disorders, and for the treatment of Amyotrophic Lateral Sclerosis or other motor neuron diseases, one or more of the disclosed neurogenesis modulating agents, with or without growth factors or other neurological agents would be delivered to the central canal. In addition to treating CNS tissue immediately surrounding a ventricle, a viral vector, DNA, growth factor, or other neurological agent can be easily administered to the lumbar cistern for circulation throughout the CNS. Infusion of EGF or similar growth factors can be used with the neurogenesis modulating agents of the invention to enhance the proliferation, migration and differentiation of neural stem cells and progenitor cells *in vivo* (see, e.g., U.S. Patent No. 5,851,832). In a preferred embodiment EGF and FGF are administered together or sequentially.

The blood-brain barrier can be bypassed by *in vivo* transfection of cells with expression vectors containing genes that code for growth factors, so that the cells themselves produce the factor. Any useful genetic modification of the cells is within the scope of the present invention. For example, in addition to genetic modification of the cells to express growth factors, the cells may be modified to express other types of neurological agents such as neurotransmitters. Preferably, the genetic modification is performed either by infection of the cells lining ventricular regions with recombinant retroviruses or transfection using methods known in the art including CaPO.sub.4 transfection, DEAE-dextran transfection, polybrene transfection, by protoplast fusion, electroporation, lipofection, and the like (see Maniatis et al., supra). Any method of genetic modification, now known or later developed can be used. With direct DNA transfection, cells could be modified by particle bombardment, receptor mediated delivery, and cationic liposomes. When chimeric gene constructs are used, they generally will contain viral, for example retroviral long terminal repeat (LTR), simian virus 40 (SV40), cytomegalovirus (CMV); or mammalian cell-specific promoters such as those for TH, DBH, phenylethanolamine N-methyltransferase, ChAT, GFAP, NSE, the NF proteins (NF-L, NF-M, NF-H, and the like) that direct the expression of the structural genes encoding the desired protein.

The uses of the invention can be used to treat any adult mammal, including humans, cows, horses, dogs, sheep, and cats. Preferably, the uses of the invention are used to treat humans. In one aspect, the invention provides a regenerative treatment for neurological disorders by stimulating adult neural stem cells to grow, proliferate, migrate, survive, and/or differentiate to replace neural cells that have been lost or destroyed. *In vivo* stimulation of such stem cells can be accomplished by locally administering a neurogenesis modulating agent of the invention to the cells in an appropriate formulation. By increasing neurogenesis, damaged or missing cells can be replaced in order to enhance blood function.

Methods for preparing the neurogenesis modulating agent dosage forms are known, or will be apparent, to those skilled in this art. The determination of an effective amount of a neurogenesis modulating agent to be administered in within the skill of one of ordinary skill in the art and will be routine to those persons skilled in the art. The amount of neurogenesis modulating agent to be administered will depend upon the exact size and condition of the patient, but will be at least 0.1. ng/kg/day, at least 1 ng/kg/day, at least 5 ng/kg/day, at least 20 ng/kg/day, at least 100 ng/kg/day, at least 0.5 ug/kg/day, at least 2 ug/kg/day, at least 5 ug/kg/day, at least 50 ug/kg/day, at least 500 ug/kg/day, at least 1 mg/kg/day, at least 5 mg/kg/day, or at least 10 mg ng/kg/day in a volume of 0.001 to 10 ml. In another method of dosage, the modulator may be administered so that a target tissue achieves a modulator concentration of 0.0001nM to 50 nM, 0.001nM to 50 nM, 0.01nM to 50 nM, 0.1nM to 50 nM, 0.1 nM to 100 nM, or at least 1 nM, at least 50 nM, or at least 100 nM. Preferred dosages include subcutaneous administration of at least 10 mg twice a week or at least 25 mg twice a week; subcutaneous administration of at least 0.04 mg/kg/week, at least 0.08 mg/kg/week, at least 0.24 mg/kg/week, at least 36 mg/kg/week, or at least 48 mg/kg/week; subcutaneous administration of at least 22 mcg twice a week or 44 mcg twice a week; or intravenous administration of at least 3-10 mg/kg once a month. Particularly preferred dosage ranges are 0.04 mg/kg to 4 mg/kg and 0.05 mg/kg to 5 mg/kg. These dosages may be increased 10x, 100x or 1000x in trasdermal or topical applications.

Pharmaceutical compositions suitable for use in the present invention include compositions wherein the active ingredients are contained in an effective amount to achieve its intended purpose. More specifically, a therapeutically effective amount means an amount effective to optimally stimulate adult stern cell proliferation. It will be appreciated that the unit content of active ingredient or ingredients contained in an individual dose of each dosage form need not in itself constitute an effective amount since the necessary effective amount can be reached by administration of a plurality of dosage units (such as capsules or tablets or combinations thereof). In addition, it is understood that at some dosage levels, an effective amount may not show any measurable effect until after a week, a month, three months, or six months of usage. Further, it is understood that an effective amount may lessen the rate of the natural deterioration that comes with age but not reverse the deterioration that has already occurred. Determination of the effective amounts is well within the capability of those skilled in the art, especially in light of the detailed disclosure provided herein. The specific dose level for any particular user will depend upon a variety of factors including the activity of the specific neurogenesis modulating agent employed, the age, the physical activity level, general health, and the severity of the disorder.

A therapeutically effective dose also refers to that amount necessary to achieve the desired effect without unwanted or intolerable side effects. Toxicity and therapeutic efficacy of a neurogenesis modulating agent can be determined by standard pharmaceutical procedures in cell cultures or experimental animals. Using standard methods, the dosage that shows effectiveness in about 50% of the test population, the ED₅₀ may be determined. Effectiveness may be any sign of adult stem cell proliferation. Similarly, the dosage that produces an undesirable side effect to 50% of the population, the SD₅₀, can be determined. Undesirable side effects include death, wounds, rashes, abnormal redness, and the like. The dose ratio between side effect and therapeutic effects can be expressed as the therapeutic index and it can be expressed as a ratio between SD₅₀/ED₅₀. Neurogenesis modulating agents with high therapeutic indexes are preferred, i.e., neurogenesis modulating agents that are effective at low dosage and which do not have undesirable side effects until very high doses. A preferred therapeutic index is greater than about 3, more preferably, the therapeutic index is greater than 10, most preferably the therapeutic index is greater than 25, such as, for example, greater than 50. Furthermore, neurogenesis modulating agents that do not have side effects at any dosage levels are more preferred. Finally, neurogenesis modulating agents that are effective at low dosages and do not have side effects at any dosage levels are most preferred. The exact formulation, route of administration and dosage can be chosen depending on the desired effect and can be made by those of skill in the art.

Dosage intervals can be determined by experimental testing. One or more neurogenesis modulating agents for use in the invention should be administered using a regimen which maintains adult stem cell proliferation at about 10% above normal, about 20% above normal, above 50% above normal such as 100% above normal, preferably about 200% above normal, more preferably about 300% above normal and most preferably about 500% above normal. In a preferred embodiment, the pharmaceutical composition for use in the invention may comprise a neurogenesis modulating agent of the invention at a concentration of between about 0.001% to about 10%, preferably between about 0.01% and about 3%, such as, for example, about 1% by weight.

Another suitable administration method is to provide a neurogenesis modulating agent of the invention through an implant or a cell line capable of expressing a neurogenesis modulating agent (e.g., peptide neurogenesis modulating agent) so that the implant or cell line can provide the neurogenesis modulating agent to a cell of the CNS.

In a preferred embodiment of the invention, the neurogenesis modulating agent of the invention induces neurogenesis in the lateral ventricle wall region of the brain. In a more preferred embodiment, the neurogenesis modulating agent induces neurogenesis in the lateral ventricle wall but not in the hippocampus.

The methods of the invention may be used to detect endogenous agents in adult neural stem cells which can be identified using RT-PCR or *in situ* hybridization techniques. In particular, genes that are up regulated or down regulated in these cells in the presence of one or more neurogenesis modulating agent of the invention can be identified. The regulation of such genes may indicate that they are involved in the mediation of signal transduction pathways in the regulation of neurogenesis function. Furthermore, by knowing the levels of expression of the these genes, and by analyzing the genetic or amino-acid sequence variations in these genes or gene products, it may be possible to diagnose disease or determine the role of stem cells in the disease. Such analysis will provide important information for using cell-based treatments for disease.

### 6. Method for Redacting a Symptom of a Disorder by Administering Cells Treated with Neuregenesis modulating agent(s)

### Harvesting Cells and Inducing Neurogenesis:

Another embodiment of the invention is directed to a method for inducing adult stern cells to undergo neurogenesis *in vitro* -- to generate large numbers of neural cells capable of differentiating into neurons, astrocytes, and oligodendrocytes. The induction of proliferation and differentiation of stem cells can be done either by culturing the cells in suspension or on a substrate onto which they can adhere. The induced cells may be used for therapeutic treatment. For example, therapy may involve, at least, (1) proliferation and differentiation of neural cells *in vitro,* then transplantation, (2) proliferation of neural cells *in vitro,* transplantation, then further proliferation and differentiation *in vivo,* (3) proliferation *in vitro,* transplantation and differentiation *in vivo,* and (4) proliferation and differentiation *in vivo*. Thus, the invention provides a means for generating large numbers of cells for transplantation into the neural tissue of a host in order to treat neurodegenerative disease and neurological trauma, for non-surgical methods of treating neurodegenerative disease and neurological trauma, and for drug-screening applications.

Stem cell progeny can be used for transplantation into a heterologous, autologous, or xenogeneic host. Multipotent stem cells can be obtained from embryonic, post-natal, juvenile or adult neural tissue, or other tissues. Human heterologous stem cells may be derived from fetal tissue following elective abortion, or from a post-natal, juvenile or adult organ donor. Autologous tissue can be obtained by biopsy, or from patients undergoing surgery (e.g., neurosurgery) in which tissue is removed, for example, during epilepsy surgery, temporal lobectomies and hippocampalectomies. Stem cells have been isolated from a variety of adult CNS ventricular regions and proliferated *in vitro* using the methods detailed herein. In various embodiments of the invention, the tissue can be obtained from any animal, including insects, fish, reptiles, birds, amphibians, mammals. The preferred source of tissue (e.g., neural tissue) is from mammals, preferably rodents and primates, and most preferably, mice and humans.

In the case of a heterologous donor animal, the animal may be euthanized, and the neural tissue and specific area of interest removed using a sterile procedure. Areas of particular interest include any area from which adult neural stern cells can be obtained that will serve to restore function to a degenerated area of the host's nervous system, particularly the host's CNS. Suitable areas include the cerebral cortex, cerebellum, midbrain, brainstem, spinal cord and ventricular tissue, and areas of the PNS including the carotid body and the adrenal medulla. Preferred areas include regions in the basal ganglia, preferably the striatum which consists of the caudate and putamen, or various cell groups such as the globus pallidus, the subthalamic nucleus, the nucleus basalis which is found to be degenerated in Alzheimer's Disease patients, or the substantia nigra pars compacta which is found to be degenerated in Parkinson's Disease patients. Particularly preferred neural tissue is obtained from ventricular tissue that is found lining CNS ventricles and includes the subependyma. The term "ventricle" refers to any cavity or passageway within the CNS through which cerebral spinal fluid flows. Thus, the term not only encompasses the lateral, third, and fourth ventricles, but also encompasses the central canal, cerebral aqueduct, and other CNS cavities.

Cells can be obtained from donor tissue (e.g., neural tissue) by dissociation of individual cells from the connecting extracellular matrix of the tissue. Tissue from a particular neural region is removed from the brain using a sterile procedure, and the cells are dissociated using any method known in the art including treatment with enzymes such as trypsin, collagenase and the like, or by using physical methods of dissociation such as with a blunt instrument. Dissociation of fetal cells can be carried out in tissue culture medium, while a preferable medium for dissociation of juvenile and adult cells is low Ca²⁺ artificial cerebral spinal fluid (aCSF). Regular aCSF contains 124 mM NaCl, 5 mM KCl, 1,3 mM MgCl₂, 2 mM CaCl₂, 26 mM NaHCO₃, and 10 mM D-glucose. Low Ca²⁺ aCSF contains the same ingredients except for MgCl₂ at a concentration of 3.2 mM and CaCl₂ at a concentration of 0.1 mM. Dissociated cells are centrifuged at low speed, between 200 and 2000 rpm, usually between 400 and 800 rpm, and then resuspended in culture medium. The cells can be cultured in suspension or on a fixed substrate. However, substrates tend to induce differentiation of the neural stem cell progeny. Thus, suspension cultures are preferred if large numbers of undifferentiated neural stem cell progeny are desired. Cell suspensions are seeded in any receptacle capable of sustaining cells, particularly culture flasks, culture plates or roller bottles, and more particularly in small culture flasks such as 25 cm² culture flasks. Cells cultured in suspension are resuspended at approximately 5 x 10⁴ to 2 x 30¹ cells/ml, preferably 1 x 10⁵ cells/ml. Cells plated on a fixed substrate are plated at approximately 2-3 x 10³ cells/cm² preferably 2.5 x 10³ cells/cm².

The dissociated neural cells can be placed into any known culture medium capable of supporting cell growth, including HEM, DMEM, RPMI, F-12, and the like, containing supplements which are required for cellular metabolism such as glutamine and other amino acids, vitamins, minerals and useful proteins such as transferrin and the like. Methods for culturing neural cells are know. See, US patents 5,980,885, 5,851,832, 5,753,506, 5,750376, 5,654,183, 5,589,376, 5,981,165 and 5,411,883. A preferred embodiment for proliferation of stem cells is to use a defined, serum-free culture medium (e.g., Complete Medium), as serum tends to induce differentiation and contains unknown components (i.e. is undefined). A defined culture medium is also preferred if the cells are to be used for transplantation purposes. A particularly preferable culture medium is a defined culture medium comprising a mixture of DMEM, F12, and a defined hormone and salt mixture. Conditions for culturing should be close to physiological conditions. The pH of the culture medium should be close to physiological pH, preferably between pH 6-8. more preferably between about pH 7 to 7.8, with pH 7.4 being most preferred. Physiological temperatures range between about 30°C to 40°C. Cells are preferably cultured at temperatures between about 32°C to about 38°C, and more preferably between about 35°C to about 37°C.

The culture medium is supplemented with at least one neurogenesis modulating agent of the invention. The number of neural stem cell progeny proliferated *in vitro* from the mammalian CNS can be increased or maintained dramatically by contacting the cell culture with a neurogenesis modulating factor of the invention. This ability to enhance the proliferation of stem cells is invaluable when stem cells are to be harvested for later transplantation back into a patient, thereby making the initial surgery 1) less traumatic because less tissue would have to be removed 2) more efficient because a greater yield of stem cells per surgery would proliferate *in vitro;* and 3) safer because of reduced chance for mutation and neoplastic transformation with reduced culture time. Optionally, the patient's stem cells, once they have proliferated *in vitro,* could also be genetically modified *in vitro* using the techniques described below. The *in vitro* genetic modification may be more desirable in certain circumstances than *in vivo* genetic modification techniques when more control over the infection with the genetic material is required.

After proliferation Stem cell progeny can be cryopreserved until they are needed by any method known in the art. In a preferred embodiment of the invention, the cells are derived from the lateral ventricle wall region of the brain. In another preferred embodiment of the invention, the cells are derived from the CNS but not from the hippocampus.

### Cellular Differentiation:

Included in the invention are methods of using the disclosed neurogenesis modulating agents to increase or maintain adult stem cell proliferation *in vitro* and obtain large numbers of differentiated cells. Differentiation of the cells can be induced by any method known in the art. In a preferred method, differentiation is induced by contacting the cell with a neurogenesis modulating agent of the invention which activates the cascade of biological events which lead to growth and differentiation. As disclosed in this invention, these events include elevation of intracellular cAMP and Ca²⁺.

Cellular differentiation may be monitored by using antibodies to antigens specific for neurons, astrocytes or oligodendrocytes can be determined by immunocytochemistry techniques well known in the art. Many neuron specific markers are known. In particular, cellular markers for neurons include NSE, NF, beta-tub, MAP-2; and for glia, GFAP (an identifier of astrocytes), galactocerebroside (GalC) (a myelin glycolipid identifier of oligodendrocytes), and the like.

Differentiation may also be monitored by *in situ* hybridization histochemistry which can also be performed, using cDNA or RNA probes specific for peptide neurotransmitter or neurotransmitter synthesizing enzyme mRNAs. These techniques can be combined with immunocytochemical methods to enhance the identification of specific phenotypes. If necessary, additional analysis may be performed by Western and Northern blot procedures.

A preferred method for the identification of neurons uses immunocytochemistry to detect immunoreactivity for NSE, NF, NeuN, and the neuron specific protein, tau-1. Because these markers are highly reliable, they will continue to be useful for the primary identification of neurons, however neurons can also be identified based on their specific neurotransmitter phenotype as previously described. Type I astrocytes, which are differentiated glial cells that have a flat, protoplasmic/fibroblast-like morphology, are preferably identified by their immunoreactivity for GFAP but not A2B5. Type II astrocytes, which are differentiated glial cells that display a stellate process-bearing morphology, are preferably identified using immunocytochemistry by their phenotype GFAP(+), A2B5(+) phenotype.

### Administration of Cells treated with a Method of the Invention:

Following in vitro expansion and neurogenesis using a method of the invention (see, Example section for a detail description of these methods), the cells can be administered to any animal with abnormal neurological or neurodegenerative symptoms obtained in any manner, including those obtained as a result of mechanical, chemical, or electrolytic lesions, as a result of experimental aspiration of neural areas, or as a result of aging processes. Particularly preferable lesions in non-human animal models are obtained with 6-hydroxy-dopamine (6-OHDA), 1-methyl-4-phenyl-1,2,3,6 tetrahydropyridine (MPTP), ibotenic acid and the like.

The methods of the instant invention allow the use of adult stem cells which are xenogeneic to the host. The methods of the invention are applied to these cells (as shown in the Examples) to expand the total number or total percent of neuronal stem cells in culture before use. Since the CNS is a somewhat immunoprivileged site, the immune response is significantly less to xenografts, than elsewhere in the body. In general, however, in order for xenografts to be successful it is preferred that some method of reducing or eliminating the immune response to the implanted tissue be employed. Thus recipients will often be immunosuppressed, either through the use of immunosuppressive drugs such as cyclosporin, or through local immunosuppression strategies employing locally applied immunosuppressants. Local immunosuppression is disclosed by Gruber, Transplantation 54:1-11 (1992). Rossini, U.S. Pat. No. 5,026,365, discloses encapsulation methods suitable for local immunosuppression.

As an alternative to employing immunosuppression techniques, methods of gene replacement or knockout using homologous recombination in embryonic stem cells, taught by Smithies et al. (Nature 317:230-234 (1985), and extended to gene replacement or knockout in cell lines (H. Zheng 35 al., PNAS, 88:8067-8071 (1991)), can be applied to precursor cells for the ablation of major histocompatibility complex (MHC) genes. Cells lacking MHC expression would allow for the grafting of enriched neural cell populations across allogeneic, and perhaps even xenogeneic, histocompatibility barriers without the need to immunosuppress the recipient. General reviews and citations for the use of recombinant methods to reduce antigenicity of donor cells are also disclosed by Gruber *(supra).* Exemplary approaches to the reduction of immunogenicity of transplants by surface modification are disclosed by Faustman WO 92/04033 (1992). Alternatively, the immunogenicity of the graft may be reduced by preparing precursor cells from a transgenic animal that has altered or deleted MHC antigens.

Grafting of cells prepared from tissue which is allogeneic to that of the recipient will most often employ tissue typing in an effort to most closely match the histocompatibility type of the recipient. Donor cell age as well as age of the recipient have been demonstrated to be important factors in improving the probability of neuronal graft survival. The efficiency of grafting is reduced with increased age of donor cells. Furthermore, grafts are more readily accepted by younger recipients compared to older recipients. These two factors are likely to be as important for glial graft survival as they are for neuronal graft survival. In some instances, it may be possible to prepare cells from the recipient's own nervous system (e.g., in the case of tumor removal biopsies, etc.). In such instances the cells may be generated from dissociated tissue and proliferated in vitro using the methods described above. Upon suitable expansion of cell numbers, the cells may be harvested, genetically modified if necessary, and readied for direct injection into the recipient's CNS.

Transplantation can be done bilaterally, or, in the case of a patient suffering from Parkinson's Disease, contralateral to the most affected side. Surgery is performed in a manner in which particular brain regions may be located, such as in relation to skull sutures, particularly with a stereotaxic guide. Cells are delivered throughout any affected neural area, in particular to the basal ganglia, and preferably to the caudate and putamen, the nucleus basalis or the substantia nigra. Cells are administered to the particular region using any method which maintains the integrity of surrounding areas of the brain, preferably by injection cannula. Injection methods exemplifed by those used by Duncan et al. J. Neurocytology, 17:351-361 (1988), and scaled up and modified for use in humans are preferred. Methods taught by Gage et al., supra, for the injection of cell suspensions such as fibroblasts into the CNS may also be employed for injection of neural precursor cells. Additional approaches and methods may be found in *Neural Grafting in the Mammalian CNS* (1985) Bjorklund and Stenevi, eds.

Although solid tissue fragments and cell suspensions of neural tissue are immunogenic as a whole, it could be possible that individual cell types within the graft are themselves immunogenic to a lesser degree. For example, Bartlett et al. (Prog. Brain Res. 82: 153-160 (1990)) have abrogated neural allograft rejection by pre-selecting a subpopulation of embryonic neuroepithelial cells for grafting by the use of immunobead separation on the basis of MHC expression. Thus, another approach is provided to reduce the chances of allo and xenograft rejection by the recipient without the use of immunosuppression techniques.

Cells when administered to the particular neural region preferably form a neural graft, wherein the neuronal cells form normal neuronal or synaptic connections with neighbouring neurons, and maintain contact with transplanted or existing glial cells which may form myelin sheaths around the neurons' axons, and provide a trophic influence for the neurons. As these transplanted cells form connections, they re-establish the neuronal networks which have been damaged due to disease and aging.

Survival of the graft in the living host can be examined using various non-invasive scans such as computerized axial tomography (CAT scan or CT scan), nuclear magnetic resonance or magnetic resonance imaging (NMR or MRI) or more preferably positron emission tomography (PET) scans. Post-mortem examination of graft survival can be done by removing the neural tissue, and examining the effected region macroscopically, or more preferably using microscopy. Cells can be stained with any stains visible under light or electron microscopic conditions, more particularly with stains which are specific for neurons and glia. Particularly useful are monoclonal antibodies which identify neuronal cell surface markers such as the M6 antibody which identifies mouse neurons. Most preferable are antibodies which identify any neurotransmitters, particularly those directed to GABA, TH, ChAT, and substance P, and to enzymes involved in the synthesis of neurotransmitters, in particular, GAD. Transplanted cells can also be identified by prior incorporation of tracer dyes such as rhodamine- or fluorescein-labelled microspheres, fast blue, bisbenzamide or retrovirally introduced histochemical markers such as the lac Z gene which produces beta galactosidase.

Functional integration of the graft into the host's neural tissue can be assessed by examining the effectiveness of grafts on restoring various functions, including but not limited to tests for endocrine, motor, cognitive and sensory functions. Motor tests which can be used include those which quantitate rotational movement away from the degenerated side of the brain, and those which quantitate slowness of movement, balance, coordination, akinesia or lack of movement, rigidity and tremors. Cognitive tests include various tests of ability to perform everyday tasks, as well as various memory tests, including maze performance.

Adult stem cells can be produced and transplanted using the above procedures to treat demyelination diseases as described in detail herein. Human demyelinating diseases for which the cells produced by the methods of the present invention may provide treatment include disseminated perivenous encephalomyelitis, MS (Charcot and Marburg types), neuromyelitis optica, concentric sclerosis, acute, disseminated encephalomyelitides, post encephalomyelitis, postvaccinal encephalomyelitis, acute hemorrhagic leukoencephalopathy, progressive multifocal leukoencephalopathy, idiopathic polyneuritis, diphtheric neuropathy, Pelizaeus-Merzbacher disease, neuromyelitis optica, diffuse cerebral sclerosis, central pontine myelinosis, spongiform leukodystrophy, and leukodystrophy (Alexander type).

Areas of demyelination in humans is generally associated with plaque like structures. Plaques can be visualized by magnetic resonance imaging. Accessible plaques are the target area for injection of neural stem cell progeny prepared by the method of the invention. Standard stereotactic neurosurgical methods are used to inject cell suspensions both into the brain and spinal cord. Generally, the cells can be obtained from any of the sources discussed above. However, in the case of demyelinating diseases with a genetic basis directly affecting the ability of the myelin forming cell to myelinate axons, allogeneic tissue would be a preferred source of the cells as autologous tissue (i.e. the recipient's cells) would generally not be useful unless the cells have been modified in some way to insure the lesion will not continue (e.g. genetically modifying the cells to cure the demyelination lesion).

Oligodendrocytes derived from cells proliferated and differentiated *in vitro* may be injected into demyelinated target areas in the recipient. Appropriate amounts of type I astrocytes may also be injected. Type I astrocytes are known to secrete PDGF which promotes both migration and cell division of oligodendrocytes (see, e.g., Nobel et al., Nature 333:560-652 (1988); Richardson et al., Cell, 53:309-319 (1988)).

A preferred treatment of demyelination disease uses undifferentiated cells (e.g., stem cells or progenitor cells). Neurospheres grown using a method of the invention can be dissociated to obtain individual cells which are then placed in injection medium and injected directly into the demyelinated target region. The cells differentiate *in vivo.* Astrocytes can promote remyelination in various paradigms. Therefore, in instances where oligodendrocyte proliferation is important, the ability of precursor cells to give rise to type I astrocytes may be useful. In other situations, PDGF may be applied topically during the transplantation as well as with repeated doses to the implant site thereafter.

Any suitable method for the implantation of cells near to the demyelinated targets may be used so that the cells can become associated with the demyelinated axons. Glial cells are motile and are known to migrate to, along, and across their neuronal targets thereby allowing the spacing of injections. Remyelination by the injection of cells is a useful therapeutic in a wide range of demyelinating conditions. It should also be borne in mind that in some circumstances remyelination by cells will not result in permanent remyelination, and repeated injections or surgeries will be required. Such therapeutic approaches offer advantage over leaving the condition untreated and may spare the recipient's life.

All the uses of this disclosure involve administration of the neurogenesis modulating agent of the invention. Administration may use known routes, including those described herein. As non-limiting examples, one or more neurogenesis modulating agents may be administered orally or by injection. The term injection, throughout this application, encompasses all forms of injection known in the art and at least the more commonly described injection methods such as subcutaneous, intraperitoneal, intramuscular, intracerebroventricular, intraparenchymal, intrathecal, and intracranial injection. Where administration is by means other than injection, all known means are contemplated including administration by through the buccal, nasal, or rectal mucosa. Commonly known delivery systems include administration by peptide fusion to enhance uptake or by via micelle or liposome delivery systems.

The uses of the invention may be tested on animal models of neurological diseases. Many such models exist. For example, they are listed in Alan A Boulton, Glen B Baker, Roger F Butterworth "Animal Models of Neurological Disease" Humana Press (1992) and Alan A Boulton, Glen B Baker, Roger F Butterworth "Animal Models of Neurological Disease II Blackwell Publishing (2000). Also, mouse models for the following diseases may be purchased by a commercial supplier such as the Jackson Laboratory: Alzheimer's Disease, Amyotrophic Lateral Sclerosis (ALS), Angelman syndrome, Astrocyte Defects, Ataxia (Movement) Defects, Behavioral and Learning Defects, Cerebellar Defects, Channel and Transporter Defects, Circadian Rhythms, Cortical Defects, Epilepsy, Fragile X Mental Retardation Syndrome, Huntington's disease, Metabolic Defects, Myclination Defects, Neural Tube Defects, Neurodegeneration, Neurodevelopmental Defects, Neuromuscular Defects, Neuroscience Mutagenesis Facility Strain, Neurotransmitter Receptor and Synaptic Vesicle Defects, Neurotrophic Factor Defects, Parkinson's Disease, Receptor Defects, Response to Catecholamines, Tremor, Tremor Defects and Vestibular and Hearing Defects. (See, e.g., http://jaxmice.jax.org/jaxmicedb/html/sbmodel 13.shtml; over 100 strains of mouse models of neurological diseases are listed in http://jaxmice.jax.org/jaxmicedb/html/model_13.shtml). Rat models of neurological diseases are numerous and may be found, for example, in recent reviews (e.g., Cenci, Whishaw and Schallert, Nat Rev Neurosci. 2002 Jul;3(7):574-9). One of skill in the art, reading this disclosure, would be able to use the results of this disclosure to design animal testing models to determine efficacy in vivo. See, also, Example 13.

Animal models of neurological diseases include, at least the following mouse strains: 129-*Akp2*^{*rm*/}*^{Sor}* 129-*Col4a3*^{*rm*/}*^{Dec}* 129-*Cstb*^{*rm*/}*^{Rm}* 129-*Edn3*^{*rm*/}*^{Ywa}* 129-*Fyn*^{*rm*/}*^{Sor}* 129-*Shh^{rm2Amc}* 129/Sv-*Csk*^{*tm*/*So*r} 129/Sv*-Kcnel*^{*tm*/}*^{Sfh}* 129/Sv-*Nog*^{*tm*/*Amc*} 129P1/ReJ 129P1/ReJ-*Lama2^{dy}* 129P3/J 129P3/JEms 129P4.Cg-*Axin^{Fu}*/+ 129P4/RrRk 129S-*Adprt1*^{*tm*/}*^{Zqw}* 129S*-Sst*^{*tm*/*Utd*} 12951-*Hprt^{tmI(cre)Mnn}* 129S1/Sv-*p*⁺ *Tyr⁺ KitI^{Si-J}*/*+* 129S1/SvImJ 129S4/SvJae-*Ntf5^{tmIJae}* 129S6-*Cln3^{tmINbm}* 129S6/SvEvTac-*Atm^{tmIAwb}* 129X1/SvJ 129X1/SvJ-*Prlr^{tmICnp}* ABP,EL*-(D2Mit132-D2Mit103*)/Frk ABP,EL*-*(*D2Mit133-D2Mit30*)/Frk ABP,EL*-*(*D2Mit422-D2Mit21*)*l*Frk ABP.EL-*El2^{e}* ABP/Le AK.B6*-Cln8^{mnd}l*+ ALR/LtJ ALS/LtJ B10.PA-*Pldn^{pa} H3^{e} a^{t}*/Sn B6 x B10.A-*H2^{a} H2-T18^{a}*/SgSnJ*-Lmx1a^{dr-7J}* B6 x B10.Q*-H2^{q}*/SgJ*-het^{2J}*/*+* B6 x B6CBCa *A^{w-J}*/*A-Myo5a*^{*f*/}*^{r} Gnb5^{flr}* B6 x BALB/cBy-*cla* B6 x BALB/cByJ-*Grid2^{Lc-J}*/+ B6 x BALB/cByJ-*Lpin1^{fld}*/+ B6 x BALB/cByJ-*mceph*/+ B6 x C57BLKS-*m Lepr^{db} Myo15^{sh2J}* B6 x STOCK *Tyr^{c-ch} Bmp5^{se} +l+ Myo6^{sv}* B6 x STOCK *a p Hps5^{ru2} Ednrb^{s}* B6 x STOCK *het* B6 x STOCK *rb* B6-*Pax3^{Sp}*.C*g-N* B6.129-Tg(Pcp2-cre)2Mpin B6.129*-Abcd1*^{*tm*/}*^{Kan}* B6.129-*Adra2c^{tmIGsb}* B6.129*-Apoe^{tmIUunc} Ldlrer^{rmIHer}* B6.129-*Ascl1^{rmIAnd}* B6.129-*Blmh^{tmIGeh}* B6.129-*Calb1^{tmIMpin}* B6.129-*DlI1^{tmIGos}* B6.129-*Gabrd^{tmIGeh}* B6.129-*Gria2^{tmIRod}* B6.129-*Grm4^{tmIHpn}* B6.129-*Grm5^{tmIRod}* B6.129-*Hdh^{tm3Mem} B6.129-Hdh^{tm4Mem}.* B6.129-*Hdh^{tm5Mem}* B6.129-*Htr2c^{tmIJul}* B6.129*-Idua^{tmIClk}* B6.129-*Kif3a^{rmIGsn}* B6.129-*Penk-rs^{tmIPlg}* B6.129*-Psen1^{tmIShn}* B6.129P1-*Lama2^{dy}* B6.129*P2(C)-Mecp2*^{*tm*/}*^{.IBird}* B6.129*P2-Apob^{tmIUne}* B6.129P2-*Apoe^{tmIUnc}* B6.129P2-*Camk2a^{tmISva}* B6.129P2-*FmrI^{tmICgr}* B6.129P2-*Hprt^{b-m3}* B6.129P2-*NcamI^{tmICgn}* B6,129P2-*Prlr^{tmICnp}* B6.129*P2-Psap^{tmISuz}* B6.129S-*Kns2^{tmIGsn}* B6.129S1-*Cnca^{tmINgor}* B6.129S1-*Grik2^{tmISfh}* B6.129S1-*Mapk10^{tmIFlv}* B6.129S1*-Thrb^{tmIDf}* B6.129S2*-Adra2a^{tmILel}* B6.129S2-*Crh^{rmIMaj}* B6.129S2-*Drd2^{tmILow}* B6.129S2-*En1^{tmIAlj}* B6.129S2*-Ntrk2^{tmIBbd}* B6.129S2-Pomc*I^{tmILow}* B6.129S3-*TwistI^{Pde}* B6.129S4*-Bdnf^{rmIJac}* B6.129S4*-Cdk5r^{tmILht}* B6.129S4-*Cdk5^{tmIKul}* B6.129S4*-Drd1a^{unIJcd}* B6.129S4*-Drd3^{tmIDac}* B6.12954.*Hdh^{tmIMem}* B6.129S4-*Hgs^{tmISor}* B6.129S4-*Ngfr^{tmIJae}* B6.129S4-*Nos1^{tmIPth}* B6.129S4-*Ntf3^{tm1Jae}* B6.129S4-*Ntf3^{tm2Jae}* B6.12954-*Pdyn^{tmIUte}* B6.129S4*-Trgpv1^{tmIJul}* B6.129S4*-Ttpa^{tmIFar}* B6.129S4-*shrm^{Gt(ROSA)53Sor}* B6.129S6*-Crebbp^{tmIDll}* B6.129S6*-Nf1^{tmIFer}* B6.129S7*-Akp2^{tmISor}* B6.129S7*-Aplp2^{tmIDbo}* B6.129S7*-App^{tmIDbo}* B6.129S7*-Chrna3^{tmIBay}* B6.129S7*-Chrna7^{tmIBay}* B6.129S7*-Ngfb^{tmIGne}*/*J* B6.129S7-*Per2^{tmIBrd}* B6.129S7*-Sod2^{tmILeb}* B6.129S7*-TwistI^{tmIBhr}* B6.129X*-Cxcr4^{tmIQmc}* B6.129X1-*Brs3^{tmIJfb}* H6.129X1-*Fos^{tmIPa}* B6.129X1-*Grpr^{tmIJfb}* B6.A-*js*/+ B6.B10Sn-*Spnb4^{qv-Ind}* B6.BKS *Ighmbp2^{nmd-2J}*/*+* B6.BKS-*Pcdhl5^{av-J}*/+ B6.BR-*Agtpbp1^{pcd}* B6.C-*H38^{c}*/By-*Kit^{W-56J}* H6.C-*H7^{b}*/By *Kit^{W-50J}* B6.C3 *Pde6b^{rdl} Hps4^{Ie}*/*+* +-*Lmx1a*^{*dr*-*J*}/+ B6.C3-Gli3*^{Xt-J}* B6.C3*-Kit^{W-44J}* B6.C3*-pi*/+ B6.C3-*stu* B6.CAST-*ahl*⁺ B6.CE-*Galc^{twl}* B6.Cg-Tg(BAC54)36Jt B6.Cg-Tg(SOD1-G93A)1Gur/J B6.Cg-TgN(SOD1)2Gur B6.Cg-TgN(SOD1-G93A)^{dl}1Gur B6.Cg-TgN(tetFosb)4468Nes B6.Cg-*Atp7a^{Mo-blo}* B6.Cg-*Atp7a^{Mo-pew2J}* B6.Cg-*Atp7a^{Mo-ro}* B6.Cg-*Cln6^{nclf}* B6.Cg*-Fbnl^{Tsk}* +/+ *Pldn^{po}* B6.Cg*-Glrb^{spa}* B6.Cg*-Kitl^{Si} Ca* B6.Cg*-Kit^{W-24J}* B6.Cg*-Kit^{W-25J}* B6.Cg*-Mitf^{Ml-nh}* B6.Cg*-Mitf^{Mi-wh}*/*Mitf^{Mi}* B6.Cg*-Mitf^{Mi-wh}* /*Mitf^{mi-rw}* B6.Cg-*Mitf^{Mi-wh}*/*Mitf^{mi-sp}* B6.Cg-*Myo7a^{shl-8J}* B6.Cg-*Os* +/+ *Cacnala^{tg-la}* B6.Cg*-Otop1^{tlt}* B6.Cg*-Pldn^{pa}* B6.Cg-*Pmp22^{Tr-J} Re*/*+* + B6.Cg*-Rora^{sg}* + +/+ *Myo5a^{d} Bmp5^{se}* B6.Cg*-Rora^{g}*/*+* B6.Cg-*T^{2J}* +/+ *Ok* B6-Cg-*Usp14^{ax-J}* B6.Cg*-enr^{Tg(MpbReg)36Pop}* B6.Cg-*wi Tyrp1^{b}*/*+ +* B6.D2*-Cacnala^{lg}* B6.D2*-Car2ⁿ*

B6.b2-*Kitl^{Si-d}*/+ B6.D2*.Kit^{W-45J}* B6.D2*-Kit^{W-73J}* B6.D2*-Pmp22^{Tr-J}* B6.D2-*ingls*/*J* and B6.KB2-*Cln8^{mnd}*/MsrJ*.* Other animal models include strains that contain the same mutations as the strains described above but in a different genetic background.

An another example, the nerogenesis modulating agents of this disclosure may be tested in the following animals models of CNS disease/disorders/trauma to demonstrate recovery, Models of epilepsia include at least electroshock-induced seizures (Billington A et al., Neuroreport 2000 Nov 27;11(17):3817-22), pentylene tetrazol (Gamaniel K et al., Prostaglandins Leukot Essent Fatty Acids 1989 Feb;35(2):63-8) or kainic acid (Riban V et al, Neuroscience 2002;112(1):101-11) induced seizures. Models of psychosis/schizophrenia include, at least, amphetamine-induced stereotypies/locomotion (Borison RL & Diamond BI, Biol Psychiatry 1978 Apr;13(2):217-25), MK-801 induced stereotypies (Tiedtke et al., J Neural Transm Gen Sect 1990;81(3):173-82), MAM (methyl azoxy ethanol- induced (Fiore M et al., Neuropharmacology 1999 Jun;38(6):857-69; Talamini LM et al., Brain Res 1999 Nov 13;847(1):105-20) or reeler model (Ballmaier M et al., Eur J Neurosci 2002 Apr;15(17):1197-205). Models of Parkinson's disease include, at least, MPTP (Schmidt &Ferger, J Neural Transm 2001;108(11):1263-82), 6-OH dopamine (O'Dell & Marshall, Neuroreport 1996 Nov 4;7(15-17):2457-61) induced degeneration. Models of Alzheimer's disease include, at least, fimbria fornix lesion model (Krugel et al., Int J Dev Neurosci 2001 Jun;19(3):263-77), basal forebrain lesion model (Moyse E et al., Brain Res 1993 Apr 2;607(1-2):154-60). Models of stroke include, at least, Focal ischemia (Schwartz DA et al., Brain Res Mol Brain Res 2002 May 30; 101(1-2):12-22); global ischemia (2- or 4-vessel occlusion) (Roof RL et al., Stroke 2001 Nov;32(11):2648-57; Yagita Y et al., Stroke 2001 Aug;32(8): 1890-6). Models of multiple sclerosis include, at least, myclin oligodendrocyte glycoprotein -induced experimental autoimmune encephalomyelitis (Slavin A et al., Autoimmunity 1998;28(2):109-20). Models of amyotrophic lateral sclerosis include, at least pmn mouse model (Kennel P et al., J Neurol Sci 2000 Nov 1;180(1-2):55-61). Models of anxiety include, at least, elevated plus-maze test (Holmes A et al., Behav Neurosci 2001 Oct;115(5):1129-44), marble burying test (Broekkamp et al., Eur J Pharmacol 1986 Jul 31;126(3):223-9), open field test (Pelleymounter et al., J Pharmacol Exp Ther 2002 Jul;302(1):145-52). Models of depression include, at least learned helplessness test, forced swim test (Shirayama Y et al., J Neurosci 2002 Apr 15;22(8):3251-61), bulbectomy (O'Connor et al., Prog Neuropsychopharmacol Biol Psychiatry 1988;12(1):41-51). Model for learning/memory include, at least, Morris water maze test (Schenk F & Morris RG, Exp Brain Res 1985;58(1):11-28). Models for Huntington's disease include, at least, quinolinic acid injection (Marco S et al., J Neurobiol 2002 Mar:50(4):323-32), transgenics/knock-ins (reviewed in Menalled LB and Chesselet MF, Trends Pharmacol Sci. 2002 Jan;23(1):32-9). Models of aged animal include, at least, the use of old animals such as old mice and old rats.

GPCRs, listed or referred to in this application, are useful as markers for specific populations of adult stem cells/progenitors, in particular aNSC/progenitors, or can serve as diagnostics. Pharmacologically active compounds (including agents for mRNA knockdown) that interact with these GPCRs or their corresponding mRNA can modulate proliferation, differentiation, survival or migration of adult stem cells/progenitors and serve as therapeutics for degenerative or psychiatric/neurological diseases, trauma or injury. In vitro they can be used to as markers to select desired cell types for transplantation. The compounds or receptors referred to in this application are useful tools in the discovery of new drugs and therapies related to stem cell proliferation, differentiation, survival and migration.

### Examples

Unless noted otherwise, all experiments were performed using standard molecular biology techniques and which is also described in copending U.S. application 10/429,062 filed May 2, 2003.

### EXAMPLE 1: Reagents

Chemicals for dissociation of tissue included trypsin, hyaluronidase and DNase (all purchased from SIGMA). Medium (DMEM 4.5 mg/ml glucose, and DMEM/F12), B27 supplement, and trypsin/EDTA were purchased from GIBCO. All plastic ware was purchased from CorningCostar. EGF for cell cultures was purchased from BD Biosciences, and the ATP-SL kit was purchased from BioThema.

For the test substances, the library was purchased from Phoenix pharmaceuticals Inc, USA, variety Pack Peptide Library (#L-001). Compounds purchased from Sigma-Aldrich included forskolin (#F6886), rolipram (#R6520), n-6, 2-o-dibutyryladenosine (#D0260), cholera toxin (#C8052), MECA (#A024), HE-NECA (#H8034), nor-Binaltorphimine (#N1771), and adrenocorticotropic hormone (#A0298).

### EXAMPLE 2: Mouse neurosphere cultures

The anterior lateral wall of the lateral ventricle of 5-6 week old mice was enzymatically dissociated in 0.8 mg/ml hyaluronidase and 0.5 mg/ml trypsin in DMEM containing 4.5 mg/ml glucose and 80 units/ml DNase at 37°C for 20 minutes. The cells were gently triturated and mixed with Neurosphere medium (DMEM/F12, B27 supplement, 12.5 mM HEPES pH7.4), 100 units/ml penicillin and 100 µg/ml streptomycin. After passing through a 70 µm strainer, the cells were pelleted at 200 x g for 4 minutes. The supernatant was subsequently removed and the cells were resuspended in Neurosphere medium supplemented with 3 nM EGF. Cells were plated out in culture dishes and incubated at 37°C. Neurospheres were ready to be split at approximately 7 days after plating.

To split neurosphere cultures, neurospheres were collected by centrifugation at 200 x g for 4 minutes. The neurospheres were resuspended in 0.5 ml trypsin/EDTA in HBSS (1x), incubated at 37°C for 2 minutes, and triturated gently to aid dissociation. Following another 3 minutes incubation at 37°C and trituration, the cells were pelleted at 220 x g for 4 minutes. Cells were resuspended in freshly prepared Neurosphere medium supplemented with 3 nM EGF and 1nM bFGF. Cells were plated out and incubated at 37°C.

### EXAMPLE 3: ATP-assay

To determine proliferation, neurospheres were split and seeded in Neurosphere medium as single cells in 96-well plates, at 10,000 cells/well. The following experiment was performed in sets of four parallel experiments (i.e., performed in quadruplicate) such that the cells may be used for different assays. Substances to be tested were added and cells were incubated at 37°C for 4 days. Cells were lysed with 0.1 % Triton-X100 in Tris-EDTA buffer. Intracellular ATP was measured using an ATP-SL kit according to the manufacturer's instructions (BioThema, Sweden). Intracellular ATP was shown to correlate with cell number. For each experiment, wells were visually examined for signs of neurogenesis and counted to confirm the results of the assay. Results were repeatable and statistically significant.

### EXAMPLE 4: cAMP detection method

For testing elevations in cAMP levels, the HitHunter EFC Cyclic AMP Chemiluminescence Assay Kit was used (DiscoveRx,USA), as purchased from Applied Biosystems. Cells were dissociated as described earlier. Cells were then seeded as non-adherent neurosphere culture at 30,000 cells/well to permit reproducible measurements of cAMP levels. The cells were allowed to rest for 2 hours prior to addition of the test substances. Following the resting period, 1 mM IBMX (3 isobutyl-1-methil-xanthine, Sigma) was added to each well and incubated for 10 minutes in 37°C, according to instructions of the manufacturer. Test substances were incubated for 20 minutes at 37°C before the cells were lysed and cAMP was measured. Each substance was tested in 3 doses (100, 10, or 1 nM), with each dose tested in quadruplicate. cAMP was measured according to kit instructions, and results were represented as pmol/well. Student's t-test was used to calculate for significance.

### EXAMPLE 5: Ca²⁺ measurement using NFAT response element reporter system

Elevations in Ca²⁺ levels were determined using a vector construct that coded for the nuclear factor of activated T cells (NFAT) response element coupled to a luciferase reporter. NFAT was previously reported to be regulated in a Ca²⁺dependent manner (Rao et al., 1997). The luciferase signal was detected with the Staedy-Glo kit (Promega). After dissociating the cells (as described above), 4-6 x 10⁶ cells were centrifuged at 250 x g for 4 minutes. The supernatant was discarded and the cells were resuspended in 100 µl Nucleofector™ Solution (Amaxa GmbH) and 10 µg NFAT-Luc vector DNA per 10⁶ cells. The suspension was transferred to a cuvette and electroporated. The transfected cells were seeded at 50,000 cells/well as non-adherent neurosphere cultures. The cells were allowed to rest over night before being contacted with the test substances. Each substance was tested in 3-4 doses (100, 15, or 1,5, 0,15 nM), with each dose tested in quadruplicate. Luciferace was measured according to the manufacturer's instructions at 18-24 hours post-induction. Results were represented as fold induction compared to untreated control. Student's t-test was used to calculate significance compared to untreated control.

### EXAMPLE 6: cDNA libraries and expression analysis

For the LVW cDNA library, RNA was isolated from the anterior lateral ventricle of adult mice (C57 black). An oligo dT-primed cDNA library was generated using standard procedures (Superscript One-Step RT-PCR with platimum Taq, Invitrogen), and then subjected to sequence analysis (9000 sequences). For the Neurosphere cDNA Library, RNA was isolated from second generation neurospheres derived from the anterior lateral ventricle wall of adult mice (C57 black), and expanded using the growth factors EGF and FGF2. An oligo dT-primed normalized cDNA library was generated using standard procedures (Superscript One-Step RT-PCR with platimum Taq, Invitrogen), and then subjected to sequence analysis (12500 sequences).

### Adult Human Neural Stem Cell (aHNSC) Cultures

A biopsy from the anterior lateral wall of the lateral ventricle was taken from an adult human patient and enzymatically dissociated in PDD (Papain 2.5U/ml; Dispase 1U/ml; Dnase I 250 U/ml) in DMEM containing 4.5 mg/ml glucose and 37°C for 20 min. The cells were gently triturated and mixed with three volumes of DMEM/F12; 10% fetal bovine serum (FBS). The cells were pelleted at 250 x g for 5 min. The supernatant was subsequently removed and the cells resuspended in DMEM F12 with 10% FBS, plated out on fibronectin coated culture dishes and incubated at 37°C in 5% CO₂. The following day the expansion of the culture was initiated by change of media to aHNSC culture media (DMEM/F12; BIT 9500; EGF 20ng/ml; FGF2 20ng/ml). The aHNSC were split using trypsin and EDTA under standard conditions. FBS was subsequently added to inhibit the reaction and the cells collected by centrifugation at 250 x g for 5 min. The aHNSC were replated in aHNSC culture media.

### RT-PCR

The cultures aHNSC were harvested and total RNA was extracted with an RNeasy mini kit (Qiagen) according to the manual.

The primer pairs for the following genes (see table below) were designed and synthesized to identify their presence in aHNSC.

| Gene name | Gene Bank Accession number | Primers |
|---|---|---|
| ADORA2A | NM_000675 | 5'-CAATGTGCTGGTGTGCTGG (SEQ ID NO:52) |
| | | 3'-TAGACACCCAGCATGAGCAG(SEQ ID NO:53) |
| EDNRA | NM_001957 | 5'-CAGGATCATTTACCAGAAC(SEQ ID NO:54) |
| | | 3'-GACGCTGCTTAAGATGTTC(SEQ ID NO:55) |
| CALCRL | NM_005795 | 5'-AGAGCCTAAGTTGCCAAAGG(SEQ ID NO:56) |
| | | 3'-GAATCAGCACAAATTCAATG(SEQ ID NO:57) |
| MC1R | NM_002386 | 5'-GAACCGGAACCTGCACTC(SEQ ID NO:58) |
| | | 3'-TGCCCAGCAGGATGGTGAG(SEQ ID NO:59) |
| MC5R | NM_005913 | 5'-GAGAACATCTTGGTCATAGG(SEQ ID NO:60) |
| | | 3'-AGCATTAAAGTGAGATGAAG(SEQ ID NO:61) |
| VIPR1 | NM_004624 | 5'-GCTACACCATTGGCTACGG(SEQ ID NO:62) |
| | | 3'-GACTGCTGTCACTCTTCCTG(SEQ ID NO:63) |
| VIPR2 | NM_003382 | 5'-GATGTCTCTTGCAACAGGAAG(SEQ ID NO:64) |
| | | 3'-GCAAACACCATGTAGTGGAC(SEQ ID NO:65) |
| SSTR1 | M_001049 | 5'-GGGAACTCTATGGTCATCTACGTGA(SEQ ID NO:66) |
| | | 3'-GAAATGTGTACAACACGAAGCCC(SEQ ID NO:67) |
| SSTR2 | NM_001050 | 5'-GGCAACACACTTGTCATTTATGTCA(SEQ ID NO:68) |
| | | 3'-AGGTAGCAAAGACAGATGATGGTGA(SEQ ID NO:69) |
| ADCYAP1R1 | NM_001118 | 5'-TACTTTGATGACACAGGCTGCT(SEQ ID NO:70) |
| | | 3-'AGTACAGCCACCACAAAGCCCT(SEQ ID NO:71) |

One step RT-PCR (Life Technologies) was performed with the primers to detect the mRNA of the genes of interest.

As a positive control primers for the gene Flt-1 were used. The gene Flt-1 is known to be expressed in the aHNSC.

As a negative control primers for the Flt-1 gene were used and Taq enzyme alone was added to ensure that the material had no genomic contamination.

The PCR products were run on an 1,5% agarose gel containing ethidium bromide. The bands of the correct size were cut out, cleaned with Qiagen's gel extraction kit. To increase the amount of material for sequencing the bands were amplified again with their corresponding primers and thereafter sequenced to confirm their identity.

### EXAMPLE 7: CREB phosphorylation assays

Briefly, NSC were split into a single cell suspension as described above. The suspension was plated in 6-well plates coated with poly-D-lysine at a density of 10⁶ cells/well. Cells were incubated in media supplemented with 1% of fetal calf serum (FBS) and allowed to adhere over night. The following morning, the media was carefully replaced with fresh DMEM/F12 and 100 nM PACAP or 100 nM cholera toxin was added to the medium. CREB phosphorylation was determined at 15 minutes and 4 hours time points after PACAP treatment, and at 15 minutes, 4 hours, 6 hours, and 8 hours time points after cholera toxin treatment. Cell lysates were collected and Western blot analysis was performed following standard procedures (Patrone et al., 1999). The specific anti-phospho-CREB antibody (1:1000 dilution; Upstate Biotechnology) was utilized.

### EXAMPLE 8: Flow cytometry analysis

Cells were split into as single cell suspensions, as described above. Cells were plated in 6-well-plates coated with poly-D-lysine at a density of 10⁶ cells/well. Following this, 1% FBS was added to the media, and the cells were allowed to adhere over night. The following morning, the media was carefully replaced with fresh DMEM/F12, and the test substance was added to a predetermined final concentration. Cells were grown for 4 days in the presence of the substance. A complete media change was performed halfway through the incubation period. Cells were harvested by incubation with trypsin/EDTA for 5 minutes at 37°C and gentle flushing with a 1000 µl pipette. Cells were flushed and centrifuged with 500 µl media at 250 x g for 4 minutes.

Following this, 2 x 10⁵ cells were transferred into minicentrifuge tubes and pelleted. The pellet was carefully resuspended in 50 µl fixation buffer (Caltag) and incubated for 15 minutes at room temperature (RT). Next, 450 µl PBS was added to the tube. The cells were centrifuged at 200 x g for 5 minutes, and the supernatant was removed. Cells were resuspended in 100 µl permeabilization buffer (Caltag) and primary antibody was added (Doublecortin 1:200, Santa Cruz) for 20 minutes at room temperature. Cells were washed as above and resuspended in secondary antibody diluted in 100 µl PBS (FITC anti-goat IgG, 1:500, Vector Laboratories). Cells were incubated in the dark for 20 minutes at room temperature. Thereafter, the cells were washed as above, resuspended in 100 µl PBS, and transferred to tubes suitable for FACS analysis.

For FACS analysis, cells were analyzed on a FACSCalibur (Becton Dickinson). Fluorescence signals from individual cells were excited by an argon ion laser at 488 nm, and the resulting fluorescence emissions from each cell was collected using bandpass filters set at 530 ± 30. Cell Quest Pro acquisition and analysis software was used to collect the fluorescence signal intensities, as well as forward and side scattering properties of the cells. The software was also used to set logical electronic gating parameters designed to differentiate between alive versus dead cells, as well as between positive and negative cells. A total of 10,000 cells per sample were analysed.

### EXAMPLE 9: cAMP levels correlate to neuronal stem cell proliferation

The aim of this investigation was to determine if cAMP and Ca²⁺ are important regulators of proliferation in adult neuronal stem cells. The experiments analyzed a large number of test substances, most of which regulated cAMP and/or Ca²⁺ via GPCRs. The results of these experiments indicated that 1) cAMP levels were correlated with mouse neural stem cells proliferation; 2) intracellular Ca²⁺ stimulation was correlated with mouse neural stem cell proliferation; and 3) adult mouse stem cells retain their potential to differentiate towards any neuronal cell (phenotype); 4) adult mouse and human neural stem cells showed similar, reproducible responses to cAMP stimulation.

To determine if cAMP pathways cause proliferation, adult neural stem cells were stimulated *in vitro* by incubation with a diverse set of cAMP cellular activators (Table 1, column 1). The results of these studies clearly demonstrate that induction of cAMP in neural stem cells leads to cell proliferation (Table 1, columns 2-6). Adult mouse stem cells grown in *vitro* were induced to proliferate following treatment with several compounds belonging to a chemical library of GPCR ligands (Example 1; Table 2, column 1). The cAMP levels were measured 15 minutes after the different treatments (Table 2, columns 5-6). ATP levels, a measure of cell number, were measured following 4 days of treatment (Table 2, columns 3-4). The results indicate a clear correlation between proliferation (ATP levels) and cAMP induction in all the substances analyzed. The GPCRs for the ligands listed in Tables 1 and 2, are shown in Table 3, columns 1-3. Expression data of the GPCRs was obtained from mouse neurospheres and lateral ventricle cDNA libraries (Table 3, columns 4-5).

**Table 1: Proliferation (ATP levels) and cAMP levels are closely correlated in mouse adult neural stem cells**

| Substance | Conc. (nMolar) | ATP (nM ATP/well) | Fold Induction ATP | cAMP (pmol/well) | Fold Induction cAMP |
|---|---|---|---|---|---|
| **Vehicle** | | **9,3±0,6** | **1,0** | **0,02±0,01** | |
| Forskolin | 1000 | 10,4±2,4 | 1,1 | 0,07±0,01 | 3,1 ** |
| Rolipram | 100 | 10,4±0,4 | 1,1 * | 0,09±0,03 | 3,8 * |
| N-6, 2-O-Dibutyryladenosine | 100 | 13,9±1,1 | 1,5 ** | 0,10±0,01 | 4,5 *** |
| Cholera toxin | 100 | 12,9±1,6 | 1,4 * | 0,07±0,01 | 3.1 *** (10 nM) |

Table 1 shows ATP levels, reflecting cell number, and cAMP levels, following adult neural stem cell treatment with cAMP chemical activators. Test substances were added to adult mouse stem cell cultures at the indicated doses, and after 15 minutes, cAMP levels were measured. ATP levels were measured after 4 days in culture. Fold induction was determined by comparison to vehicle treated cells. Data was represented as the mean ± SD value of quadruplicate tests in a typical experiment. The representative values were calculated based on two separate experiments. *, P<0.05; **, P<0.005; *** P<0,001 (Student's t test). n.s. = non significant.

**Table 2: GPCR ligands that stimulate proliferation (ATP levels) and cAMP activation in mouse adult neural stem cells. Each one of these agents is a neurogenesis modulating agent.**

| Substance | Conc. (nM) | ATP (nM ATP/well) | Fold Induction ATP | cAMP (pmol /well) | Fold Induction cAMP |
|---|---|---|---|---|---|
| **Vehicle** | | **16,4±1,3** | | **2,23±0,52** | |
| Adrenocorticotropic hormone | 10 | 18,6±1,0 | 1,1 * | 6,36±2,58 | 2,8 * (100 nM) |
| **Vehicle** | | **16,4±1,3** | | **1,84±0,53** | |
| Endothelin-1 (human, porcine) | 10 | 41,7±7,2 | 2,5 * | 3,64±1,13 | 2.0 * |
| **Vehicle** | | **4,5±0,6** | | **1,84±0,53** | |
| MECA | 100 | 7,4±0,7 | 1,6 ** | 3,89±1,00 | 2,1 * |
| HE-NECA | 1000 | 8,2±1,1 | 1,8 ** | 3,32±0,28 | 1,8 *** (10nnM) |
| **Vehicle** | | **8,6±1,4** | | **0,13±0,02** | |
| [Cys3,6, Tyr8, Pro9]-Substance P | 100 | 11,2±0,4 | 1,3 ** | 0,29±10 | 2,2 * |
| **Vehicle** | | **8,6±1,4** | | **0,13±0,02** | |
| [D-Arg0, Hyp3, I15, D-Ig17, Oic8]-Bradykinin | 100 | 13,1±2,1 | 1,5 * | 0,17±0,02 | 1,3 * (10nM) |
| **Vehicle** | | **10,3±0,6** | | **0,06±0,01** | |
| Adrenomedullin (human) | 100 | 11,6±0,8 | 1,1 * | 0,15±0,3 | 2,5 ** |
| **Vehicle** | | **8,8±0,9** | | **0,03±0,01** | |
| [Des-Arg9,Leu8]-Bradykinin | 10 | 9,8±0,4 | 1,1 * | 0,09±0,02 | 2,6 * (1nMn |
| [Des-Arg9]-Bradykinin | 1 | 10,4±1,0 | 1,2 * | 0,06±0,01 | 1,7 *** |
| [D-Pen2-5]-Enkephalin | 10 | 10,7±0,9 | 1,2 ** | 0,06±0,01 | 1,7 * |
| [D-pGlul, D-Phe2, D-Trp3,6]-LH-RH | 100 | 11,1±0,4 | 1,3 *** | 0,07±0,02 | 2,0*(1nM) |
| **Vehicle** | | **7,8±2,0** | | **0,21±0,08** | |
| Adrenomedullin (26-52) | 1 | 11,4±0,7 | 1,5 ** | 0,33±0,07 | 1,6 * |
| Adrenomedullin (22-52) | 100 | 12,3±1,1 | 1,6 ** | 0,34±0,07 | 1,6 * |
| α-Neo-Endorphin | 100 | 13,8±2,1 | 1,8 ** | 0,36±0,09 | 1,7 * (1nM) |
| **Vehicle** | | **10,3±2,2** | | **0,17±0,04** | |
| β-MSH | 100 | 13,6±1,6 | 1,3 * | 0,23 ±0,02 | 1,3 ** (10nM) |
| **Vehicle** | | **7,8±2,0** | | **2,23±0,52** | |
| α-MSH | 100 | 14,7±3,5 | 1,9*** | 5,82±0,86 | 2,6 ** (100nM) |
| **Vehicle** | | **7,1±0,5** | | **0,17±0,04** | |
| Thyrocalcitonin (Salmon) | 1 | 9,2±0,7 | 1,3 * | 0,63±0,23 | 3,8*(1nM) |
| **Vehicle** | | **7,1±0,5** | | **0,10±0,02** | |
| Calcitonin | 100 | 9,9±1,6 | 1,4* | 0,35±0,15 | 3,3 * |
| (human) | | | | | |
| CART (61-102)□ | 100 | 8,3±0,4 | 1,2 ** | 0,₁3±0,02 | 1,2 * (10nM) |
| **Vehicle** | | **8,8±0,9** | | **0,09±0,03** | |
| Cholecystokinin Octapeptide [CCK(26-33)] | 10 | 9,8±0,4 | 1,1 * | 0,27±0,06 | 3,1 ** (100nM) |
| **Vehicle** | | **7,6±1,0** | | **0,14±0,02** | |
| DTLET | 10 | 9,2±0,9 | 1,2 * | 0,20±0,02 | 1,4 * (100nM) |
| **Vehicle** | | **7,6±1,0** | | **0,14±0,02** | |
| DDAVP | 100 | 11,5±1,4 | 1,5 * | 0,27±0,02 | 1,9 *** (10nM) |
| **Vehicle** | | **8,5±1,5** | | **0,84±0,11** | |
| Eledoisin | 100 | 10,4±1,1 | 1,2 * | 1,0±0,06 | 1,2 * (1nM) |
| **Vehicle** | | **6,3±0,2** | | **0,57±0,14** | |
| γ-MSH | 10 | 7,4±0,5 | 1,2 * | 0,96±0,18 | 1,7 * (100nM) |
| **Vehicle** | | **8,7±1,5** | | **0,05±0,06** | |
| α-Neurokinin | 100 | 11,0±1,4 | 1,3 * | 0,11±0,03 | 2,3 * (10nM) |
| **Vehicle** | | **9,4±1,4** | | **0,03±0,01** | |
| PACAP-38 | 100 | 26.9±3,7 | 2,9 ** | 0,13±0,03 | 4,2 ** |
| **Vehicle** | | **10,3±2,2** | | **0,17±0,04** | |
| Beta-ANP | 100 | 13,6±2,1 | 1,3 * | 070±0,04 | 4,2 *** |
| **Vehicle** | | **6,3±0,2** | | **0,57±0,14** | |
| Galanin (1-13)-Spantide-Amide, M40 | 100 | 7,10±0,5 | 1,1 * | 0,82±0,08 | 1,4 ** (1nM) |
| **Vehicle** | | **12,5±1,8** | | **0,07±0,06** | |
| [Sar9, Met (0)11]-Substance P | 100 | 39,7±2,1 | 3,2 *** | 0,16±0,05 | 2,2 * |
| **Vehicle** | | **12,5±1,8** | | **0,30±0,08** | |
| Sarafotoxin S6a | 10 | 43,3±4,5 | 3,5 *** | 0,41±0,06 | 1,4 * |
| **Vehicle** | | **15,2±3,2** | | **0,07±0,06** | |
| Sarafotoxin S6b | 100 | 43,0±7,8 | 2,8 ** | 0,43±0,22 | 6,0 * |
| Sarafotoxin S6c | 10 | 39,9±6,6 | 2,6 ** | 0,21±0,03 | 3,0 ** |
| **Vehicle** | | **13,5±1,9** | | **0,06±0,01** | |
| [Nle8,18,Tyr34]-Parathyroid Hormone (1-34) Amide (Human) | 1000 | 23,5±2,7 | 1,7 ** | 0,16±0,05 | 2,6 * (10nM) |
| ACTH (Human) | 1000 | 15,7±1,3 | 1,2 * | 0,11±0,02 | 1,8 ** (100nM) |
| Glucagon-Like Peptide-1 (7-37) (Human) | 1000 | 18,3±1,4 | 1,3 ** | 0,08±0,01 | 1,4* (100nM) |
| **Vehicle** | | **12,3±1,1** | | **0,14±0,05** | |
| Exendin-3 | 100 | 14,2±1,0 | 1,2 * | 0,21±0,03 | 1,5 * (10nM) |
| **Vehicle** | | **12,3±1,1** | | **0,30±0,08** | |
| Exendin-4 | 1000 | 16,0±2,0 | 1,3 * | 0,49±0,04 | 1,6 *** (10nM) |
| **Vehicle** | | **12,3±1,1** | | **0,20±0,07** | |
| Urotensin II (Globy) | 100 | 14,3±1,1 | 1,2 * | 0,50±0,15 | 2,6 * (10nM) |
| Vasoactive Intestinal Peptide (Human, Porcine, Rat) | 1000 | 20,6±1,2 | 1,7 *** | 0,39±0,12 | 2,0 * (100nM) |
| **Vehicle** | | **13,4±1,8** | | **0,97±0,46** | |
| Nor-Binaltorphimine | 0,1 | 19,4±3,2 | 1,4 * | 6,10±3,72 | 6,3 ** (0,01nM) |
| **Vehicle** | | **7,8±2,0** | | **0,21±0,08** | |
| Agouti Related Protein (87-132)-Amide (Human) | 10 | 11.2±1,7 | 1,4 * | 0,5±0,20 | 2,4 * |

Table 2 shows ATP levels, reflecting cell number, and cAMP levels. Test substances were added to adult mouse stem cell cultures at the indicated doses. After four days, values for ATP and cAMP were assayed. Fold induction was determined by comparison to vehicle treated cells. Data was represented as the mean ± SD value of quadruplicate tests in a typical experiment. The representative values were based on two separate experiments. *, P<0.05; **, P<0.005; *** P<0,001 (Student's t test). n.s. = non significant. ^{a} Significant in lower concentration.

**Table 3: Expression analysis of possible targets for the GPCR ligands listed in Table 2**

| Official Name | Locus Link Symbol mouse | Locus Link Symbol human | Mouse neurosphere Expression | Mouse lateral ventricular wall expression | Human neurosphere Expression |
|---|---|---|---|---|---|
| Adenosine A2a receptor | Adora2a | ADORA2A | YES | YES | n.d. |
| Adenosine A2b receptor | Adora2b | ADORA2B | YES | YES | YES |
| Adenosine A3 receptor | Adora3 | ADORA3 | n.d. | n.d. | n.d. |
| Adenylate cyclase activating polypeptide 1 receptor 1 | Adcyap1r1 | ADCYAP1R 1 | YES | YES | YES |
| Adrenomedulli n receptor | Admr | ADMR | n.d. | n.d. | YES |
| arginine vasopressin receptor 2 | Avpr2 | AVPR2 | n.d. | n.d. | n.d. |
| Bradykinin receptor, beta 1 | Bdkrb1 | BDKRB1 | n.d. | n.d. | n.d. |
| Bradykinin receptor, beta | Bdkrb2 | BDKRB2 | n.d. | n.d. | n.d. |
| Calcitonin receptor | Calcr | CALCR | n.d. | n.d. | n.d. |
| Calcitonin receptor-like | Calcrl | CALCRL | n.d. | n.d. | YES |
| Cholecystokini n A receptor | Cckar | CCKAR | n.d. | n.d. | YES |
| Cholecystokini n B receptor | Cckbr | CCKBR | n.d. | n.d. | YES |
| Endothelin receptor type A | Ednra | EDNRA | YES | YES | YES |
| Endothelin receptor type B | Ednrb | EDNRB | YES | YES | n.d. |
| Galanin receptor 1 | Galrl | GALR1 | n.d. | n.d. | n.d. |
| Galanin receptor 2 | Galr2 | GALR2 | n.d. | n.d. | n.d. |
| Galanin receptor 3 | Galr3 | GALR3 | n.d. | n.d. | n.d. |
| Glucagon-like peptide 1 receptor | Glplr | GLP1R | n.d. | n.d. | n.d. |
| Gonadotropin | Gnrhr | GNRHR | n.d. | n.d. | n.d. |
| releasing hormone receptor | | | | | |
| Melanocortin 1 receptor | Mc1r | MC1R | n.d. | n.d. | YES |
| Melanocortin 2 receptor | Mc2r | MC2R | n.d. | n.d. | n.d. |
| Melanocortin 3 receptor | Mc3r | MC3R | n.d. | n.d. | n.d. |
| Melanocortin 4 receptor | Mc4r | MC4R | n.d. | n.d. | n.d. |
| Melanocortin 5 receptor | Mc5r | MC5R | n.d. | n.d. | YES |
| Natriuretic peptide receptor 1 | Npr1 | NPR1 | n.d. | n.d. | n.d. |
| Natriuretic peptide receptor 2 | Npr2 | NPR2 | n.d. | n.d. | n.d. |
| Natriuretic peptide receptor 3 | Npr3 | NPR3 | n.d. | n.d. | n.d. |
| Opioid receptor, delta 1 | Oprdl | OPRD1 | n.d. | n.d. | n.d. |
| Opioid receptor, kappa 1 | Oprkl | OPRK1 | n.d. | n.d. | n.d. |
| Tachykinin receptor 1 | Tacr1 | TACR1 | n.d. | n.d. | n.d. |
| Tachykinin receptor 2 | Tacr2 | TACR2 | n.d. | n.d. | n.d. |
| Tachykinin receptor 3 | Tacr3 | TACR3 | n.d. | n.d. | n.d. |
| Vasoactive intestinal peptide receptor 1 | Vipr1 | VIPR1 | YES | YES | YES |
| Vasoactive intestinal peptide receptor 2 | Vipr2 | VIPR2 | YES | YES | YES |
| G protein-coupled receptor 14 | Gpr14 | GPR14 | n.d. | n.d. | n.d. |
| Parathyroid hormone | Pthrl | PTHR1 | n.d. | n.d. | n.d. |
| receptor 1 | | | | | |

Table 3 shows that GPCRs were found to be expressed in adult mouse and/or human stem cell cultures. Gene expression in mouse cells or tissue was determined by cDNA library analysis, and human expression using RT-PCR.

A number of compounds that were not previously identified as enhancers of intracellular cAMP were tested for stimulation of neurogenesis. This test was used to determine: 1) if there were additional compounds that could stimulate neurogenesis by any mechanism; and 2) if there were additional compounds that could stimulate neurogenesis by increasing intracellular cAMP. Surprisingly, several of these compounds were found to stimulate neurogenesis even though they were not previously known increase intracellular cAMP levels. The compounds screened included: (Des-Arg9,Leu8)-Bradykinin, (Des-Arg9)-Bradykinin, Alpha-NeoEndorphin, CART (61-102), DTLET, Eledoisin, Urotensin II, [Nle8,18, Tyr34]-Parathyroid Hormone (1-34) Amide, and [Cys3,6, Tyr8, Pro9]-Substance P (see Table 2). Our review of the literature showed that these properties (of elevating intracellular cAMP, and inducing neurogenesis) were not previously know.

The experiments were repeated with visual examination of the wells for signs of neurogenesis and to confirm the results of the previous assay. The results were repeatable. The visual analysis confirmed our previous findings and did not reveal anything that would contradict the previous findings.

### EXAMPLE 10: Ca²⁺ levels correlate to neuronal stem cell proliferation

To show that proliferation upon intracellular Ca²⁺increase in response to GPCR ligands is upregulated in adult mouse stem cells grown *in vitro,* the cells were treated with a number of test substances (Table 4, column 1). Ca²⁺ was measured via regulation of the nuclear factor of activated T cells gene (NFAT; Example 5). The results showed a clear correlation between ATP levels (Table 4, columns 3-4) and NFAT up-regulation (Table 4, columns 5-6). This indicates that Ca²⁺ levels are strongly correlated with neural stem cells proliferation. The GPCRs that trigger Ca²⁺ for the ligands analyzed (Table 5, columns 1-3) were found to be present in the two cDNA libraries analyzed (Example 6; Table 5, columns 4-5). Tables 3 and 5 (columns 6) indicate GPCRs that were identified in human stem cells material using RT-PCR analysis. This corroborates our findings in adult mouse stem cells suggesting that the activation of Ca²⁺can also be important for triggering GPCR-mediated proliferation in human stem cells.

**Table 4: GPCR ligands that regulate NFAT-Luciferace reporter (Ca²⁺) and ATP (proliferation). Each one of these agents is a neurogenesis modulating agent.**

| **Substance** | **Conc** (nMolar) | **ATP** (nM ATP/well) | **Fold Induction** ATP | **NFAT Lucifarece units** | **Fold Induction** NFAT |
|---|---|---|---|---|---|
| **Vehicle** | | **9,8±2,1** | | **42,9±7,4** | |
| Amylin Receptor Antagonist/Calcitonin(8-32) | 100 | 15,0±2,2 | 1,5 * | 57,3±5,4 | 1,3 *** (0,15nM) |
| **Vehicle** | | **9,8±1,6** | | **42,9±7,4** | |
| ANP (human) | 10 | 12,7±1,0 | 1,3 * | 65,9±8,9 | 1,5 * (1,5nM) |
| Vehicle | | 8,8±0,9 | | 21,1±4,1 | |
| CGRP (8-37) | 100 | 10,4±0,5 | 1,2 ** | 28,3±1,1 | 1,3 ** (at 15nM) |
| **Vehicle** | | **4,5±0,6** | | **3,4±0,8** | |
| Endothelin-1 (human, Bovine, Canine, Mouse, Porcine, Rat) | 10 | 14,4±2,4 | 3,2 * | 8,3±2,5 | 2,4 * (0,15nM) |
| **Vehicle** | | **6,3±0,2** | | **2,4±1,4** | |
| γ-MSH | 10 | 7,4±0,5 | 1,2 * | 4,8±1,5 | 2,0 * (1,5nM) |
| **Vehicle** | | **7,5±0,6** | | **2,4±1,4** | |
| Growth Hormone Releasing Factor | 10 | 12,6±0,9 | 1,7 * | 4,5±0,6 | 1,9 ** (15nM) |
| **Vehicle** | | **8,2±0,8** | | **2,4±1,4** | |
| MGOP 27 | 100 | 10,2±1,2 | 1,2 * | 4,0±0,4 | 1,7 * (1,5nM) |
| **Vehicle** | | **9.4±1,4** | | **3,5±0,9** | |
| PACAP-38 | 10 | 22,0±0,9 | 2,3 *** | 6,2±1,6 | 1,7 * |
| **Vehicle** | | **12,5±1,8** | | **1,9±0,5** | |
| Sarafotoxin S6a | 1 | 38,9±3,2 | 3,1 *** | 6,3±2,4 | 3,4 * |
| **Vehicle** | | **15,2±3,2** | | **1,9±0,5** | |
| Sarafotoxin S6b | 100 | 43,0±7,8 | 2,8 ** | 13,4±7,0 | 7,2 * |
| Sarafotoxin S6c | 1 | 41,6±4,8 | 2,7 *** | 8,3±2,0 | 4,4 * |
| Septide | 100 | 25,1±3,1 | 1,7 * | 3,7±0,9 | 2,0 * |
| **Vehicle** | | **14,0±1,8** | | **1,9±0,5** | |
| Somatostatin-28 | 10 | 17,1±1,5 | 1,2 * | 3,0±0,4 | 1,6 * (100nM) |
| **Vehicle** | | **9,3±0,06** | | **8,2±0,7** | |
| Cholera toxin from Vibrio Cholerae | 100 | 12,9±1,6 | 1,4 * | 11,6±1,0 | 1,4 ** |
| **Vehicle** | | **9,8±2,1** | | **5.6±0.5** | |
| Angiotensin II (human synthetic) | | 11,7±0,6 | 1,2 * | 12.0±3.7 | 2,1 * |
| **Vehicle** | | **8,8±0,9** | | **5.6±0.5** | |
| [D-Pen2-5]-Enkephalin | 10 | 10,7±0,9 | 1,2 * | 10.3±2.1 | 1,8* (100nM) |
| **Vehicle** | | **10,3±0,6** | | **5.6±0.5** | |
| Adrenomedullin | 100 | 11,6±0,8 | 1,1 * | 12.4±0.9 | 2,2 ** |
| Endothelin-1 (human, Porcine,) | 10 | 35,3±3,7 | 1,3 * | 13,3±1,3 | 1,6 ** |

Table 4: Adult mouse neuronal stem cells were transiently transfected with NFAT-Luciferace construct and induced with test substances at the indicated doses. Cells were analyzed 24 hours after induction. NFAT-Luciferace activity and ATP was analyzed. Fold induction was determined by comparison to vehicle treated cells. The data was represented as the mean ± SD value of quadruplicate tests in a typical experiment. The representative values were based on two separate experiments. *, P<0.05; **, P<0.005; *** P<0,001 (Student's t test). n.s. = non significant. ^{a} Significant in lower concentration.

**Table 5: Expression analysis of targets for the GPCR ligands listed in Table 4**

| **Official Name** | **Locus Link Symbol mouse** | **Locus Link Symbol human** | Mouse neurosphere expression | Mouse lateral ventricular wall expression | Human neurosphere expression |
|---|---|---|---|---|---|
| Adenylate cyclase activating polypeptide 1 receptor 1 | Adcyaplrl | ADCYAP1R1 | YES | YES | YES |
| Angiotensin receptor 1b | Agtr1b | AGTR1 | n.d. | n.d. | n.d. |
| Angiotensin II receptor, type 2 | Agtr2 | AGTR2 | n.d. | n.d. | n.d. |
| Calcitonin receptor | Calcr | CALCR | n.d. | n.d. | n.d. |
| Calcitonin receptor-like | Calcrl | CALCRL | n.d. | n.d. | YES |
| Endothelin receptor type A | Ednra | EDNRA | YES | YES | YES |
| Endothelin receptor type B | Ednrb | EDNRB | YES | YES | n.d. |
| Growth hormone releasing hormone receptor | Ghrhr | GHRHR | n.d. | n.d. | n.d. |
| Melanocortin 1 receptor | Mc1r | MC1R | n.d. | n.d. | YES |
| Melanocortin 3 receptor | Mc3r | MC3R | n.d. | n.d. | n.d. |
| Melanocortin 4 receptor | Mc4r | MC4R | n.d. | n.d. | n.d. |
| Melanocortin 5 receptor | Mc5r | MC5R | n.d. | n.d. | YES |
| Natriuretic peptide receptor 1 | Npr1 | NPR1 | n.d. | n.d. | n.d. |
| Natriuretic peptide receptor 2 | Npr2 | NPR2 | n.d. | n.d. | n.d. |
| Natriuretic peptide receptor 3 | Npr3 | NPR3 | n.d. | n.d. | n.d. |
| Opioid receptor, delta 1 | Oprdl | OPRD1 | n.d. | n.d. | n.d. |
| Somatostatin receptor 1 | Sstr1 | SSTR1 | YES | YES | YES |
| Somatostatin receptor 2 | Sstr2 | SSTR2 | YES | YES | YES |
| Somatostatin receptor 3 | Sstr3 | SSTR3 | YES | YES | n.d. |
| Somatostatin receptor 4 | Sstr4 | SSTR4 | YES | YES | n.d. |
| Somatostatin receptor 5 | Sstr5 | SSTR5 | YES | YES | n.d. |
| Tachykinin receptor 1 | Tacr1 | TACR1 | n.d. | n.d. | n.d. |
| Vasoactive intestinal peptide receptor 1 | Vipr1 | VIPR1 | YES | YES | YES |
| Vasoactive intestinal peptide receptor 2 | Vipr2 | VIPR2 | YES | YES | YES |

### EXAMPLE 11: Human and mouse stem cell responses to cAMP stimulation

The experiments described above suggest that intracellular induction of cAMP occurs in proliferative mouse adult neural stem cells. To further investigate the relevance of these findings, the cAMP pathway was studied in human and mouse systems. Since CREB phosphorylation is a well known downstream effector in the cAMP activation pathway (Lonze and Ginty, 2002), the phosphorylation state of this transcription factor was investigated in time course experiments. Two cAMP activators, PACAP and cholera toxin, were utilized (Example 7). PACAP and cholera toxin were added to the adult human and mouse neuronal stem cells. Western blot analysis showed similar up-regulation in mouse as in human neuronal stem cells (FIG. 1). The results clearly demonstrate that the pattern of CREB phosphorylation in both systems is responsive to PACAP and cholera toxin in a reproducible manner (FIG. 1). This suggests that mouse and human stem cells respond in similar ways following cAMP cell induction. GPCRs for which ligands were shown to be proliferative in mouse aNSCs were present also in human aNSCs (Table 3, column 6)

### EXAMPLE 12: Adult neural stem cells retain their neuronal potential following GPCRs proliferative stimuli.

In order to understand if proliferating adult neural stem cells retained their neuronal potential following GPCR ligand treatment, analysis was performed to determine the expression of the early neuronal marker Doublecortin. Neural stem cells were treated with several GPCRs ligands for 4 day. Flow cytometric analysis was performed on the cells with an antibody against the early neuronal marker Doublecortin. As shown in Table 6, all GPCR ligand-treated cells analysed continued to express Doublecortin after four days in culture (see also Example 8). This indicated that the ligand-treated adult NSCs were still able to differentiate towards a neuronal phenotype.

**Table 6: Adult neural stem cells retain their neuronal potential after proliferation with GPCR ligands.**

| Substance | Concentration | % Doublecortin-positive cells | Fold Induction |
|---|---|---|---|
| **EGF/FGF** | **3nM/1nM** | **2.63±1.86** | **1** |
| Forskolin | 10µM | 6.3 | 2.5 |
| Cholera toxin from Vibrio Cholerae | 100nM | 6.7 | 2.6 |
| Endothelin I, human, porcine | 10nM | 5.0 | 2.0 |
| PACAP-38 | 100nM | 5.2 | 2.0 |
| (D-Trp7,Ala8,D-Phe10)-α-Melanocyte stimulating hormone F:6-11/GHRP | 100nM | 5.3 | 2.1 |
| α-Neurokinin | 100nM | 4.6 | 1.8 |
| Thyrocalcitonin salmon | 100nM | 3.9 | 1.5 |
| MECA | 10 µM | 2.2 | 0.9 |
| [Des-Arg9]-Bradykinin | 100nm | 4.5 | 1.8 |
| Eledoisin | 100nM | 4.3 | 1.7 |
| γ-Melanocyte stimulating hormone | 100nM | 4.1 | 1.6 |
| [D-Pen2-5]-Enkephalin | 100nM | 3.3 | 1.3 |
| α-Neo-Endorphin (Porcine) | 100nM | 4.0 | 1.6 |
| DTLET | 100nM | 4.1 | 1.6 |
| [D-Arg0, Hyp3, Ig15, D-Igl7, Oic8]-Bradykinin | 100nM | 3.6 | 1.4 |
| [D-pGlu1, D-Phe2, D-Trp3,6]-LH-RH | 100nM | 3.4 | 1.3 |
| Adrenomedullin (Human) | 100nM | 4.2 | 1.6 |
| Adrenomedullin (22-52) (Human) | 100nM | 2.0 | 0.8 |
| Agouti Related Protein (87-132)-Amide (Human) | 100nM | 2.4 | 0.9 |
| Angiotensin II (Human) | 100nM | 3.1 | 1.2 |
| β-Melanocyte Stimulating Hormone | 100nM | 4.1 | 1.6 |
| CART (61-102)(Human, Rat) | 100nM | 4.7 | 1.8 |
| Cholecystokinin Octapeptide [CCK(26-33)](Non-sulfated) | 100nM | 3.2 | 1.3 |
| DDAVP (enhances human learning and memory) | 100nM | 4.6 | 1.8 |
| Sarafotoxin S6a (cardiotoxin isotoxin) | 100nM | 3.2 | 1.3 |

### Table 6: Cells proliferated by GPCR ligands maintained or increased their potential to mature towards a neuronal phenotype.

The sum of these results and previous studies on PACAP (see, e.g, U.S. Patent Application Serial No. 60/377,734 filed May 3, 2002; U.S. Patent Application Serial No. 06/393,264, filed July 2, 2002; U.S. Patent Application Serial No. 10/429,062, filed May 2, 2003; Mercer et al, J. Neurosci. Res.*,* manuscript in press) indicate that compounds (e.g., natural ligands, small chemical entities, affinity proteins, etc.) that increase levels of cAMP or Ca²⁺ can stimulate proliferation of adult neural stem cells *in vitro* and *in vivo.* In some cases, this stimulation may be mediated by GPCRs. In addition, cAMP elevation alone (i.e., in a GPCR-independent-manner) can elicit an increase in the proliferation of neural stem cells. This increase was observed with various cAMP activators, including 1) cAMP-derivatives such as N-6,2-O-Dibutyryladenosine; 2) inhibitors of cAMP phosphodiesterases such as 3-Isobutyl-1-Methylxanthine (IBMX) and rolipram; 3) adenylate cyclase activators such as forskolin; and 4) compounds that elevate ADP-ribosylation of the alpha-subunit of the stimulatory G protein (Gs), such as cholera toxin. Cholera toxin and related compounds are believed to act by reducing GTPase activity and activating the alpha-subunit. This leads to an increase in the activity of adenylate cyclase resulting in increased levels of cAMP. Further, as shown herein, several ligands that act through GPCRs and increase the intracellular Ca²⁺ content, are also effective in promoting neurogenesis, including cellular proliferation.

These experiments show that cAMP or Ca²⁺ activation can be used in therapeutic approaches to modulate proliferation, differentiation, survival or migration of adult neural stem cells/progenitor cells in different physiological or pathological conditions. The various compounds (e.g., GPCRs ligands) described herein may display different cellular specificities and fate profiles, which make them suited for different physiological and pathological conditions. Importantly, adult neural stem cells retained their neuronal potential following GPCR ligand treatment. The sum of these findings indicates a broad range of therapeutic compounds for stimulating neurogenesis through the intracellular elevation of cAMP and/or C_{a2+.}

### Example 13 Progenitor Cell Proliferation

A neurogenesis modulating agent is administered intraperitoneally to adult test animals (n=12) at various concentrations from 0.01 to 100 mg/kg. Saline is given as a negative control. Starting two hours after neurogenesis modulating agent administration, animals are injected with four intraperitoneal injections of bromodeoxyuridine (BrdU; 50 mg/kg each) at three hour intervals. Animals are perfused after 1, 2 or 3 days or after 1, 2, 3 or 4 weeks after neurogenesis modulating agent administration. For animals studied for more than one day BrdU is administered by minipump.

In perfusion, animals are perfused transcardially with 50 ml of ice cold phosphate buffered saline (PBS) and then 100 ml of 4% paraformaldehyde in PBS. Brains are fixed after removal in 4% paraformaldehyde in PBS for 24 hours at 4 C for at least 3 days before sectioning. Sections are prepared using a freezing microtome and stored in cyroprotectant at - 20 C before immunostaining for BrdU.

Sections are immunostained for BrdU with mouse anti-BrdU paired with a biotinylated goat anti mouse IgG. Avidin-biotin-horseratish peroxidase (HRP) complex is applied to sections and immunoreactivity are visualized by reacting diaminobenzidine with the HRP. Standard techniques are used to estimate the total number of BrdU positive cells in each section and in each region of the brain.

Analysis and quantification is performed for proliferative brain regions, migratory streams and areas of clinical relevance (some, but not all, of these areas are exemplified below). These analysis is performed with DAB (diamine benzidine) or fluorescence visualisation using one or several of the following antibodies: as neuronal markers NeuN, Tuj1, anti-tyrosine hydroxylase, anti-MAP-2 etc.; as glial markers anti-GFAP, anti-S100 etc.; as oligodendrocyte markers anti-GalC, anti-PLP etc. For BrdU visualisation: anti-BrdU. Quantification will be performed in all areas of the brain using stereological quantification.

In particular, the following regions are of particular interest: dorsal hippocampus dentate gyrus, dorsal hippocampus CA1/alveus, olfactory bulb (OB), subventricular zone (SVZ) and striatum. Quantification of double-staining with confocal microscope will be performed for every structure (e.g., OB, DG, CA1/alveus, SVZ, wall-to-striatum) checking BrdU+ for double-staining with the lineage markers.

Other experimental details not listed here are known to one of skill in the art and may be found, for example, in Pencea V et al., J. Neurosci Sept 1 (2001), 21(17):6706-17

The experiment is performed with wildtype animals as well as an animal model of a neurological disease. Such models are enumerated in the detailed discussion section. One preferred animal is the mouse.

Throughout this specification, various patents, published application, GenBank DNA and protein sequences, and scientific references are cited to describe the state and content of the art.

### REFERENCES

Altman J, Das G (1965) Autoradiographic and histological evidence of postnatal hippocampal neurogenesis in rats. J Comp Neurol 124:319-335.
Altman J, Das G (1967) Postnatal neurogenesis in the guinea-pig. Nature 214:1098-1101. BiebI M, Cooper CM, Winkler J. Kuhn HG (2000) Analysis of neurogenesis and programmed cell death reveals a self- renewing capacity in the adult rat brain. Neurosci Lett 291:17-20.
Craig CG, Tropepe V, Morshead CM, Reynolds BA, Weiss S, van der Kooy D (1996) In vivo growth factor expansion of endogenous subependymal neural precursor cell populations in the adult mouse brain. J Neurosci 16:2649-2658.
Doetsch F, Caille I, Lim DA, Garcia-Verdugo JM, Alvarez-Buylla A (1999) Subventricular zone astrocytes are neural stem cells in the adult mammalian brain. Cell 97:703-716.
Gage FH, Kempermann G, Palmer TD, Peterson DA, Ray J (1998) Multipotent progenitor cells in the adult dentate gyrus. J Neurobiol 36:249-266.
Herman JP, Abrous ND (1994) Dopaminergic neural grafts after fifteen years: results and perspectives. Prog Neurobiol 44:1-35.
Jacobson M (1991) Histosenesis and morphogenesis of cortical structures. In: Developmental Neurobiology, pp 401-451: Plenum Press, New York.
Johansson CB, Svensson M, Wallstedt L, Janson AM, Frisen J (1999a) Neural stem cells in the adult human brain. Exp Cell Res 253:733-736.
Johansson CB, Momma S, Clarke DL, Risling M, Lendahl U, Frisen J (1999b) Identification of a neural stem cell in the adult mammalian central nervous system. Cell 96:25-34.
Johe KK, Hazel TG, Muller T, Dugich-Djordjevic MM, McKay RD (1996) Single factors direct the differentiation of stem cells from the fetal and adult central nervous system. Genes Dev 10:3129-3140.
Kuhn HG, Winkler J, Kempermann G, Thal LJ, Gage FH (1997) Epidermal growth factor and fibroblast growth factor-2 have different effects on neural progenitors in the adult rat brain. J Neurosci 17:5820-5829.
Lois C, Alvarez-Buylla A (1993) Proliferating subventricular zone cells in the adult mammalian forebrain can differentiate into neurons and glia. Proc Natl Acad Sci U S A 90:2074-2077.
Lonze BE, Ginty DD (2002) Function and regulation of CREB family transcription factors in the nervous system. Neuron 35:605-623.
Magavi SS, Leavitt BR, Macklis JD (2000) Induction of neurogenesis in the neocortex of adult mice [see comments]. Nature 405:951-955.
McKay R (1997) Stem cells in the central nervous system. Science 276:66-71.
Nakatomi H, Kuriu T, Okabe S, Yamamoto S, Hatano O, Kawahara N, Tamura A, Kirino T, Nakafuku M (2002) Regeneration of hippocampal pyramidal neurons after ischemic brain injury by recruitment of endogenous neural progenitors. Cell 110:429-441.
Neves SR, Ram PT, Iyengar R (2002) G protein pathways. Science 296:1636-1639.
Palmer TD, Markakis EA, Willhoite AR, Safar F, Gage FH (1999) Fibroblast growth factor-2 activates a latent neurogenic program in neural stem cells from diverse regions of the adult CNS. J Neurosci 19:8487-8497.
Patrone C, Andersson S, Korhonen L, Lindholm D (1999) Estrogen receptor-dependent regulation of sensory neuron survival in developing dorsal root ganglion. Proc Natl Acad Sci U S A 96:10905-10910.
Pencea V, Bingaman KD, Wiegand SJ, Luskin MB (2001) Infusion of Brain-Derived Neurotrophic Factor into the Lateral Ventricle of the Adult Rat Leads to New Neurons in the Parenchyma of the Striatum, Septum, Thalamus, and Hypothalamus. J Neurosci 21:6706-6717.
Rajan P, McKay RD (1998) Multiple routes to astrocytic differentiation in the CNS. J Neurosci 18:3620-3629.
Rao A, Luo C, Hogan PG (1997) Transcription factors of the NFAT family: regulation and function. Annu Rev Immunol 15:707-747.
Snyder EY, Yoon C, Flax JD, Macklis JD (1997) Multipotent neural precursors can differentiate toward replacement of neurons undergoing targeted apoptotic degeneration in adult mouse neocortex. Proc Natl Acad Sci U S A 94:11663-11668.
Williams BP, Park JK, Alberta JA, Muhlebach SG, Hwang GY, Roberts TM, Stiles CD (1997) A PDGF-regulated immediate early gene response initiates neuronal differentiation in ventricular zone progenitor cells. Neuron 18:553-562.
Zhao M, Momma S, Delfani K, Carlen M, Cassidy RM, Johansson CB, Brismar H, Shupliakov O, Frisen J, Janson AM (2003) Evidence for neurogenesis in the adult mammalian substantia nigra. Proc Natl Acad Sci U S A 100:7925-7930.

## Claims

1. At least one agent that elevates intracellular cAMP levels in neural tissue, wherein said agent is selected from the group consisting of Thyrocalcitonin, Calcitonin, Glucagon-Like Peptide-1 (7-37), Exendin-3 and Exendin-4, and analogs of Glucagon-Like Peptide-1 (7-37), Exendin-3 or Exendin-4, wherein said Glucagon-Like Peptide-1 (7-37), Exendin-3 or Exendin-4 analog interacts with a G-protein coupled receptor (GPCR) which is the Glucagon-like peptide 1 receptor, and a combination thereof, or a cAMP analog, wherein said cAMP analog is selected from the group consisting of 8-pCPT-2-O-Me-cAMP, 8-Br-cAMP, Rp-cAMPS, 8-Cl-cAMP, Dibutyryl cAMP, pCPT-cAMP, and N6-monobutyryladenosine 3',5'-cyclic monophosphate, for use in increasing neurogenesis in neural tissue of a patient exhibiting a central nervous system disorder selected from the group consisting of neurodegenerative disorders, ischemic disorders, neurological traumas, and learning and memory disorders, wherein the agent increases neurogenesis in the patient, thereby increasing neurogenesis in the neural tissue of the patient, and wherein increasing neurogenesis is increasing proliferation, differentiation, migration or survival of an adult neural stem cell in said neural tissue.

2. The agent of claim 1 wherein the central nervous system disorder is **characterised by** at least one symptom selected from the group consisting of tension, abnormal movements, abnormal behaviour, tics, hyperactivity, combativeness, hostility, negativism, memory defects, sensory defects, cognitive defects, hallucinations, acute delusions, poor self-care, withdrawal and seclusion.

3. The agent of any claim 1 or claim 2 wherein the nervous system disorder is selected from the group consisting of Parkinson's disease and Parkinsonian disorders, Huntington's disease, Alzheimer's disease, multiple sclerosis, amyotrophic lateral sclerosis, Shy-Drager syndrome, progressive supranuclear palsy, Lewy body disease, spinal ischemia, ischemic stroke, cerebral infarction, spinal cord injury, and cancer-related brain and spinal cord injury, multi-infarct dementia, geriatric dementia and cognitive defects.

4. The agent of any one of claims 1 to 3 wherein the agent is administered to the central nervous system of the patient.

5. The agent of any one claims 1 to 4 wherein the agent is administered by a route selected from the group consisting of oral, subcutaneous, intraperitoneal, intramuscular, intracerebroventricular, intraparenchymal, intrathecal, intracranial, buccal, mucosal, nasal, and rectal administration,

6. The agent of any one of claims 1 to 5 wherein the agent is administered by a liposome delivery system.

7. The agent of any one of claims 1 to 6 wherein said increasing neurogenesis is performed by an activation of a GPCR receptor in said neural tissue, wherein said GPCR receptor is selected from the group consisting of Calcitonin receptor, Calcitonin receptor-like receptor and Glucagon-like peptide 1 receptor.

8. An *in vitro* method for increasing or stimulating the intracellular levels of cAMP in an adult neural stem cell, comprising contacting said cell with an effective amount of an agent selected from the group consisting of Thyrocalcitonin, Calcitonin, Glucagon-Like Peptide-1 (7-37), Exendin-3 and Exendin-4, and a combination thereof, whereby the intracellular level of cAMP in said cell is increased, and wherein proliferation, differentiation, migration or survival of said adult neural stem cell is increased.

9. A method for increasing neurogenesis *in vitro* comprising the steps of
a) culturing a population of neural cells comprising adult neural stem cells;
b) adding to the cultured cells at least one neurogenesis increasing agent;
c) if necessary, repeating step b) until a desired level of neurogenesis is achieved,
wherein increasing neurogenesis is increasing proliferation, differentiation, migration or survival of said adult neural stem cells, and further wherein said neurogenesis increasing agent is as defined in claim 1.

10. The method of claim 9 wherein the adult stem cell is isolated from tissue selected from the group consisting of cortex, olfactory tubercle, retina, septum, lateral ganglionic eminence, medial ganglionic eminence, amygdala, hippocampus, thalamus, hypothalamus, ventral and dorsal mesencephalon, brain stem, cerebellum, spinal cord,

11. The method of claim 9 or claim 10 wherein the adult stem cell is isolated from a mammal.

12. The method of claim 11 wherein the mammal is a human.

13. Use of at least one agent that elevates intracellular cAMP levels in neural tissue, wherein said agent is as defined in claim 1, in the manufacture of a composition for increasing neurogenesis in neural tissue of a patient exhibiting a central nervous system disorder selected from the group consisting of neurodegenerative disorders, ischemic disorders, neurological traumas, and learning and memory disorders, wherein the agent increases neurogenesis in the patient, thereby increasing neurogenesis in the neural tissue of the patient, and wherein increasing neurogenesis is increasing proliferation, differentiation, migration or survival of an adult neural stem cell in said neural tissue.

## Patentansprüche

1. Wenigstens ein Wirkstoff, der intrazelluläre cAMP-Pegel in Nervengewebe anhebt, wobei der Wirkstoff ausgewählt ist aus der Gruppe, die besteht aus Thyrocalcitonin, Calcitonin, glucagonartiges Peptid-1 (7-37), Exendin-3 und Exendin-4 und Analoga von glucagonartigem Peptid-1 (7-37), Exendin-3 oder Exendin-4, wobei das Analogon von glucagonartigem Peptid-1 (7-37), Exendin-3 oder Exendin-4 mit einem mit G-Protein gekoppelten Rezeptor (GPCR), der der glucagonartige Peptid-1-Rezeptor ist, wechselwirkt, und aus einer Kombination hiervon oder aus einem cAMP-Analogon, wobei das cAMP-Analogon aus der Gruppe gewählt ist, die besteht aus 8-pCPT-2-OMe-cAMP, 8-Br-cAMP, Rp-cAMPS, 8-Cl-cAMP, Dibutyryl-cAMP, pCPT-cAMP und 3',5'-zyklischem N6-Monobutyryladenosin-Monophosphat für die Verwendung bei der Erhöhung der Neurogenese in Nervengewebe eines Patienten, der eine Störung des zentralen Nervensystems zeigt, die ausgewählt ist aus der Gruppe, die besteht aus neurodegenerativen Störungen, ischämischen Störungen, neurologischen Traumata sowie Lern- und Gedächtnisstörungen, wobei der Wirkstoff die Neurogenese in dem Patienten erhöht, um **dadurch** die Neurogenese im Nervengewebe des Patienten zu erhöhen, und wobei die Erhöhung der Neurogenese eine Erhöhung der Vermehrung, der Differenzierung, der Migration oder des Überlebens einer adulten neuronalen Stammzelle in dem Nervengewebe ist.

2. Wirkstoff nach Anspruch 1, wobei die Störung des zentralen Nervensystems **gekennzeichnet ist durch** wenigstens ein Symptom, das ausgewählt ist aus der Gruppe, die besteht aus Spannung, anomalen Bewegungen, anomalem Verhalten, Muskelzucken, Hyperaktivität, Streitsucht, Feindseligkeit, Negativismus, Gedächtnisdefekten, Wahrnehmungsdefekten, kognitiven Defekten, Halluzinationen, akuten Wahnvorstellungen, geringer Selbstsorge, Entzug und Zurückgezogenheit.

3. Wirkstoff nach Anspruch 1 oder Anspruch 2, wobei die Störung des Nervensystems aus der Gruppe ausgewählt ist, die besteht aus der Parkinson-Krankheit und Parkinsonschen Störungen, der Huntington-Krankheit, der Alzheimer-Krankheit, der Multiplen Sklerose, der amyotrophischen lateralen Sklerose, dem Shy-Drager-Syndrom, der progressiven supranuklearen Lähmung, der Lewykörper-Krankheit, der Wirbelsäulen-Ischämie, dem ischämischen Schlag, der zerebralen Infarktbildung, der Rückenmarksverletzung und der krebsbezogenen Hirn- und Rückenmarksverletzung, der Mehrfachinfarkt-Demenz, der geriatrischen Demenz und kognitiven Defekten.

4. Wirkstoff nach einem der Ansprüche 1 bis 3, wobei der Wirkstoff für das zentrale Nervensystem des Patienten verabreicht wird.

5. Wirkstoff nach einem der Ansprüche 1 bis 4, wobei der Wirkstoff auf einem Weg verabreicht wird, der ausgewählt ist aus der Gruppe, die besteht aus einer oralen, einer subkutanen, einer intraperitonealen, einer intramuskulären, einer intrazerebroventrikulären, einer intraparenchymalen, einer intrathezalen, einer intracranialen, einer buccalen, einer mukosalen, einer nasalen und einer rektalen Verabreichung.

6. Wirkstoff nach einem der Ansprüche 1 bis 5, wobei der Wirkstoff durch ein Liposom-Verabreichungssystem verabreicht wird.

7. Wirkstoff nach einem der Ansprüche 1 bis 6, wobei die zunehmende Neurogenese durch eine Aktivierung eines GPCR-Rezeptors in dem Nervengewebe ausgeführt wird, wobei der GPCR-Rezeptor aus der Gruppe ausgewählt ist, die besteht aus einem Calcitoninrezeptor, einem calcitoninrezeptorartigen Rezeptor und einem glucagonartigen Peptid-1-Rezeptor.

8. In vitro-Verfahren für die Erhöhung oder Stimulation der intrazellulären Pegel von cAMP in einer adulten Nervenstammzelle, das das Herstellen eines Kontakts zwischen der Zelle und einer wirksamen Menge eines Wirkstoffs umfasst, der ausgewählt ist aus der Gruppe, die besteht aus Thyrocalcitonin, Calcitonin, glucagonartigem Peptid-1 (7-37), Exedin-3 und Exedin-4 und einer Kombination hiervon, wobei der intrazelluläre Pegel von cAMP in der Zelle erhöht wird und wobei die Vermehrung, Differenzierung, Migration oder das Überleben der adulten Nervenstammzelle erhöht wird.

9. Verfahren zum in vitro-Erhöhen der Neurogenese, das die folgenden Schritte umfasst:
a) Kultivieren einer Population von Nervenzellen, die adulte Nervenstammzellen enthalten;
b) Hinzufügen wenigstens eines die Neurogenese erhöhenden Wirkstoffs zu den kultivierten Zellen;
c) gegebenenfalls Wiederholen des Schrittes b), bis ein bestimmter Grad der Neurogenese erzielt ist,
wobei die Erhöhung der Neurogenese eine Erhöhung der Vermehrung, der Differenzierung, der Migration oder des Überlebens der adulten Nervenstammzellen ist und wobei ferner der Neurogeneseerhöhungswirkstoff wie in Anspruch 1 definiert beschaffen ist.

10. Verfahren nach Anspruch 9, wobei die adulte Stammzelle aus Gewebe isoliert wird, das gewählt ist aus der Gruppe die besteht aus Kortex, olfaktorischem Tuberkel, Retina, Septum, lateraler ganglionischer Eminenz, medialer ganglionischer Eminenz, Amygdala, Hypocampus, Thalamus, Hypothalamus, ventrales und dorsales Mesencephalon, Hirnstamm, Cerebellum, Rückenmark.

11. Verfahren nach Anspruch 9 oder Anspruch 10, wobei die adulte Stammzelle aus einem Säugetier isoliert wird.

12. Verfahren nach Anspruch 11, wobei das Säugetier ein Mensch ist.

13. Verwendung wenigstens eines Wirkstoffs, der intrazelluläre cAMP-Pegel in Nervengewebe anhebt, wobei der Wirkstoff wie in Anspruch 1 definiert geschaffen ist, bei der Herstellung einer Zusammensetzung für die Erhöhung der Neurogenese im Nervengewebe eines Patienten, der eine Störung des zentralen Nervensystems zeigt, die ausgewählt ist aus der Gruppe, die besteht aus neurodegenerativen Störungen, ischämischen Krankheiten, neurologischen Traumata und Lern- und Gedächtnisstörungen, wobei der Wirkstoff die Neurogenese in dem Patienten erhöht, wodurch die Neurogenese in dem Nervengewebe des Patienten erhöht wird; und wobei die Erhöhung der Neurogenese eine Erhöhung der Vermehrung, Differenzierung, Migration oder des Überlebens einer adulten Nervenstammzelle in dem Nervengewebe ist.

## Revendications

1. Au moins un agent qui élève des niveaux intracellulaires de cAMP dans un tissu neural, ledit agent étant choisi parmi le groupe constitué de la Thyrocalcitonine, de la Calcitonine, du Glucagon-Like Peptide-1 (7-37), de l'Exendine-3 et de l'Exendine-4, et d'analogues du Glucagon-Like Peptide-1 (7-37), de l'Exendine-3 et de l'Exendine-4, dans lequel ledit analogue du Glucagon-Like Peptide-1 (7-37), de l'Exendine-3 et de l'Exendine-4 interagit avec un récepteur couplé aux protéines G (GPCR) qui est le récepteur du Glucagon-Like Peptide-1, et une combinaison de ceux-ci, ou un analogue du cAMP, dans lequel ledit analogue du cAMP est choisi parmi le groupe constitué de 8-pCPT-2-0-Me-cAMP, 8-Br-cAMP, Rp-cAMPS, 8-Cl-cAMP, Dibutyryl cAMP, pCPT-cAMP et monophosphate de N6-monobutyryladénosine 3',5'-cyclique, pour une utilisation dans l'augmentation de la neurogénèse dans le tissu neural d'un patient présentant un trouble du système nerveux central choisi parmi le groupe constitué de troubles neurodégénératifs, de troubles ischémiques, de traumatismes neurologiques et de troubles d'apprentissage et de mémoire, dans lequel l'agent augmente la neurogénèse chez le patient, en augmentant ainsi la neurogénèse dans le tissu neural du patient, et dans lequel la neurogénèse croissante augmente la prolifération, la différenciation, la migration ou la survie d'une cellule souche neurale adulte dans ledit tissu neural.

2. Agent selon la revendication 1, dans lequel le trouble du système nerveux central est **caractérisé par** au moins un symptôme choisi parmi le groupe constitué de tension, de mouvements anormaux, d'un comportement anormal, de tics, d'hyperactivité, de combativité, d'hostilité, de négativisme, de défauts mémoriels, de défauts sensoriels, de défauts cognitifs, d'hallucinations, d'illusions aigues, de manque de soins, de repli sur soi et de solitude.

3. Agent selon l'une quelconque des revendications 1 ou 2, dans lequel le trouble du système nerveux est choisi parmi le groupe constitué de la maladie de Parkinson et des troubles parkinsoniens, de la maladie de Huntington, de la maladie d'Alzheimer, de la sclérose en plaques, de la sclérose latérale amyotrophique, du syndrome de Shy-Drager, de l'ophtalmoplégie supranucléaire progressive, de la maladie du corps de Lewy, de l'ischémie spinale, de l'accident vasculaire cérébral ischémique, de l'infarctus cérébral, d'une lésion médullaire, et d'une lésion cérébrale et médullaire liée à un cancer, de la démence vasculaire, de la démence gériatrique et des défauts cognitifs.

4. Agent selon l'une quelconque des revendications 1 à 3, l'agent étant administré au système nerveux central du patient.

5. Agent selon l'une quelconque des revendications 1 à 4, l'agent étant administré par une voie choisie parmi le groupe constitué d'une administration orale, sous-cutanée, intrapéritonéale, intramusculaire, intracérébroventriculaire, intraparenchymale, intrathécale, intracrânienne, buccale, muqueuse, nasale et rectale.

6. Agent selon l'une quelconque des revendications 1 à 5, l'agent étant administré par un système de délivrance de liposomes.

7. Agent selon l'une quelconque des revendications 1 à 6, dans lequel ladite neurogénèse croissante est réalisée par une activation d'un récepteur GPCR dans ledit tissu neural, dans lequel ledit récepteur GPCR est choisi parmi le groupe constitué du récepteur de la Calcitonine, du récepteur du Calcitonine receptor-like et du récepteur du Glucagon-Like Peptide-1.

8. Procédé in vitro pour augmenter ou stimuler les niveaux intracellulaires de cAMP dans une cellule souche neurale adulte, comportant les étapes consistant à mettre ladite cellule en contact avec une quantité efficace d'un agent choisi parmi le groupe constitué de la Thyrocalcitonine, de la Calcitonine, du Glucagon-Like Peptide-1 (7-37), de l'Exendine-3 et de l'Exendine-4, et d'une combinaison de ceux-ci, de sorte que le niveau intracellulaire de cAMP dans ladite cellule est augmenté, et dans lequel la prolifération, la différenciation, la migration ou la survie de ladite cellule souche neurale adulte est augmentée.

9. Procédé pour augmenter la neurogénèse in vitro, comportant les étapes consistant à :
a) mettre en culture une population de cellules neurales comportant des cellules souches neurales adultes ;
b) ajouter aux cellules mises en culture au moins un agent augmentant la neurogénèse ;
c) si nécessaire, répéter l'étape b) jusqu'à ce qu'un niveau voulu de neurogénèse soit obtenu,
dans lequel la neurogénèse croissante augmente la prolifération, la différenciation, la migration ou la survie desdites cellules souches neurales adultes, et également dans lequel ledit agent augmentant la neurogénèse est tel que défini dans la revendication 1.

10. Procédé selon la revendication 9, dans lequel la cellule souche adulte est isolée du tissu choisi parmi le groupe constitué du cortex, du tubercule olfactif, de la rétine, du septum, de l'éminence ganglionnaire latérale, de l'éminence ganglionnaire médiale, de l'amygdale, de l'hippocampe, du thalamus, de l'hypothalamus, du mésencéphale ventral et dorsal, du tronc cérébral, du cervelet et de la moelle épinière.

11. Procédé selon la revendication 9 ou 10, dans lequel la cellule souche adulte est isolée d'un mammifère.

12. Procédé selon la revendication 11, dans lequel le mammifère est un être humain.

13. Utilisation d'au moins un agent qui élève des niveaux intracellulaires de cAMP dans un tissu neural, dans lequel ledit agent est tel que défini dans la revendication 1, dans la fabrication d'un composition destinée à augmenter la neurogénèse dans le tissu neural d'un patient présentant un trouble du système nerveux central choisi parmi le groupe constitué de troubles neurodégénératifs, de troubles ischémiques, de traumatismes neurologiques et de troubles d'apprentissage et de mémoire, dans lequel l'agent augmente la neurogénèse chez le patient, en augmentant ainsi la neurogénèse dans le tissu neural du patient, et dans lequel la neurogénèse croissante augmente la prolifération, la différenciation, la migration ou la survie d'une cellule souche neurale adulte dans ledit tissu neural.
